(19) Europäisches Patentamt
European Patent Office
Office européen des brevets

(11) EP 1 242 586 B1

(12) **EUROPEAN PATENT SPECIFICATION**

(45) Date of publication and mention of the grant of the patent:
**13.10.2010 Bulletin 2010/41**

(21) Application number: **00988824.9**

(22) Date of filing: **21.12.2000**

(51) Int Cl.:
***C12N 9/12*** *(2006.01)* ***C12Q 1/70*** *(2006.01)*

(86) International application number:
**PCT/FI2000/001135**

(87) International publication number:
**WO 2001/046396 (28.06.2001 Gazette 2001/26)**

(54) **RNA POLYMERASES FROM BACTERIOPHAGE PHI 6-PHI 14 AND USE THEREOF**

RNA-POLYMERASEN AUS BACTERIOPHAGEN PHI6 - PHI14 UND DEREN ANWENDUNGEN

POLYMERASES D'ARN DU BACTERIOPHAGE PHI 6-PHI 14 ET UTILISATION

(84) Designated Contracting States:
**AT BE CH CY DE DK ES FI FR GB GR IE IT LI LU MC NL PT SE TR**

(30) Priority: **21.12.1999 FI 992751**

(43) Date of publication of application:
**25.09.2002 Bulletin 2002/39**

(60) Divisional application:
**09171411.3**

(73) Proprietor: **RNA-Line Oy**
**FIN-04430 Järvenpää (FI)**

(72) Inventors:
- **Makeyev, Eugeny**
  **FIN-00250 Helsinki (FI)**
- **Bamford, Dennis**
  **FIN-04430 Järvenpää (FI)**

(74) Representative: **Sundman, Patrik Christoffer et al**
**Seppo Laine Oy**
**Itämerenkatu 3 B**
**00180 Helsinki (FI)**

(56) References cited:
**WO-A-00/36112 WO-A-91/10746**

- **LEONARD MINDICH ET AL: "Nucleotide Sequence of the large double-stranded RNA segment of bacteriophage phi 6: genes specifying the viral replicase and transcriptase" JOURNAL OF VIROLOGY, vol. 62, no. 4, 1988, pages 1180-1185, XP002901667**
- **JUUTI JARMO T ET AL: "Structure and NTPase activity of the RNA-translocating protein (P4) of bacteriophage phi6" J. MOL BIOL. , vol. 279, 1998, pages 347-359, XP002901668**
- **JUUTI J T ET AL: "Protein P7 of phage phi6 RNA polymerase complex, acquiring of RNA packaging activity by in Vitro assembly of the purified protein onto deficient particles" J. MOL. BIOL., vol. 266, 1997, XP002901669**
- **PATTON J T ET AL: "Genome replication and packaging of segmented double-stranded RNA viruses" VIROLOGY, vol. 277, 2000, XP002901670**
- **MINDICH LEONARD : "Reverse genetics of dsRNA bacteriophage phi6" ADVANCED IN VIRUS RESEARCH, vol. 53, 1999, XP002901671**
- **MINDICH LEONARD: "Precise packaging of the three genomic segments of the double-stranded-RNA bacteriophage phi6" MICROBIOLOGY AND MOLECULAR BIOLOGY REVIEWS, vol. 63, no. 1, 1999, XP002901672**
- **GOTTLIEB, P. ET AL.: "In vitro replication, packaging, and transcription of the segmented double-stranded RNA genome of bacteriophage phi 6: studies with procapsids assembled from plasmid-encoded proteins." JOURNAL OF BACTERIOLOGY, vol. 172, no. 10, October 1990 (1990-10), pages 5774-5782,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 242 586 B1

SEQUENCE LISTING

<110> Makeyev, Eugeny Bamford, Dennis

<120> A novel polymerase protein for RNA synthesis and use thereof

<130> hy1

<140>
<141>

<150> 19992751
<151> 1999-12-21

<160> 8

<170> PatentIn Ver. 2.1

<210> 1
<211> 1998
<212> DNA
<213> bacteriophage f6 of Pseudomonas syringae

<400> 1

```
atgccgagga gagctcccgc gttccctctg agcgatatca aggctcagat gctgttcgca 60
aataacatca aggcccaaca agcctcgaag cgtagcttca aagagggggc gattgaaacg 120
tacgaagggc tgctttcagt agaccctcgg tttttgagtt tcaagaacga gctctctcgg 180
tatctgaccg accacttccc ggcgaacgtc gacgagtatg gtcgtgttta tggaaacggt 240
gttcgtacca acttctttgg tatgcgccac atgaacgggt ttccaatgat ccccgcgacg 300
tggccactcg cttccaacct taagaaacgt gccgacgctg acctagccga tggccctgtt 360
tctgagcgcg acaatctact ctttcgcgcc gcagtccggc ttatgttttc agatctagag 420
cctgttccgc tgaagatccg taaaggatcg tcaacctgca tcccgtattt ttctaacgat 480
atgggaacga agatcgagat cgccgagcgc gctcttgaga aagcggaaga agctggcaat 540
ctgatgctgc aaggtaagtt tgatgacgcc taccagctcc accaaatggg tggtgcctat 600
tacgtcgtgt atcgtgcaca atcgaccgat gctatcacac tcgaccctaa gaccggaaaa 660
ttcgtgtcaa aggatcgtat ggtcgctgac ttcgaatacg cagtcacggg cggtgagcaa 720
ggctcgctgt tcgctgcttc gaaggatgcc tctcgtttga aggaacagta cgggatagat 780
gtcccggacg ggtttttctg cgagcggcgt cgtaccgcta tgggtggtcc gttcgcgttg 840
aacgctccta tcatggccgt tgcgcaacct gtgcgaaaca aaatttactc caagtacgct 900
tacacctttc accatactac tcgtcttaat aaggaggaaa aggtgaaaga gtggtcgttg 960
tgcgtcgcta ctgacgtatc cgaccacgac acgttctggc ctggatggct gcgggatctc 1020
atctgtgatg aactgctcaa catggggtac gctccgtggt gggttaagtt gttcgagacc 1080
tcgctcaaac tgcccgttta cgtgggcgct cctgctcctg agcagggcca cacgttgttg 1140
ggtgatccgt ccaaccctga tctcgaagtt ggtctctcgt ccggacaagg ggcgaccgac 1200
ctcatgggca cgttgctcat gagtatcacc tacctggtga tgcaacttga tcacaccgct 1260
cctcacctca acagtcgaat caaggacatg ccatcagcat gccgctttct tgactcgtat 1320
tggcaaggac acgaggagat ccgtcagatc tcaaaatctg atgatgctat gcttggctgg 1380
accaaaggtc gtgctttggt tggtggtcat cgtttgttcg agatgctgaa agagggtaag 1440
gttaacccct caccttacat gaagatctcc tacgagcacg gtggcgcctt ccttggtgac 1500
atcctgcttt acgactcgcg tcgtgagcct ggctctgcca tcttcgttgg taacatcaac 1560
tcaatgctga acaaccagtt cagccctgag tacggtgtcc aatcgggcgt tcgcgaccga 1620
tctaagcgca aacggccgtt ccccggtctt gcttgggcgt cgatgaaaga tacctacggt 1680
gcctgtccga tctactctga tgtgctggag gcgatcgagc gttgctggtg gaacgcgttc 1740
ggtgagtcgt accgtgcgta tcgtgaagat atgcttaaac gcgacactct cgaactatca 1800
cgctacgttg cgtcgatggc tcgtcaagcc gggctggctg aactcactcc cattgatttg 1860
gaggtgcttg ctgacccgaa caaactccag tataagtgga ccgaggccga tgtctcggcg 1920
aatatccacg aggtactgat gcatggcgta tcggtcgaaa agactgagcg ctttctccgt 1980
tctgtaatgc ctaggtaa                                               1998
```

<210> 2
<211> 24
<212> DNA
<213> Artificial Sequence

<220>
<223> Description of Artificial Sequence:oligonucleotide

<400> 2
ggtaagcgcc atatgccgag gaga 24

<210> 3

<211> 33
<212> DNA
<213> Artificial Sequence

<220>
<223> Description of Artificial Sequence:oligonucleotide

<400> 3
tacgaattcc ggcatgatta cctaggcatt aca 33

<210> 4
<211> 23
<212> DNA
<213> Artificial Sequence

<220>
<223> Description of Artificial Sequence: oligonucleotide

<400> 4
ggataaacaa gtccttgtat aac 23

<210> 5
<211> 18
<212> DNA
<213> Artificial Sequence

<220>
<223> Description of Artificial Sequence:oligonucleotide

<400> 5
cgttcagttc tcagttct 18

<210> 6
<211> 24
<212> DNA
<213> Artificial Sequence

<220>
<223> Description of Artificial Sequence: oligonucleotide

<400> 6
ggtaagcgcc atatgccgag gaga 24

<210> 7
<211> 38
<212> DNA
<213> Artificial Sequence

<220>
<223> Description of Artificial Sequence:oligonucleotide

<400> 7
ctgaattcta atacgactca ctatagatcc gaccgtag 38

<210> 8
<211> 665
<212> PRT
<213> bacteriophage f6 of Pseudomonas syringae

<400> 8

```
Met Pro Arg Arg Ala Pro Ala Phe Pro Leu Ser Asp Ile Lys Ala Gln
1               5                   10                  15

Met Leu Phe Ala Asn Asn Ile Lys Ala Gln Gln Ala Ser Lys Arg Ser
            20                  25                  30

Phe Lys Glu Gly Ala Ile Glu Thr Tyr Glu Gly Leu Leu Ser Val Asp
        35                  40                  45

Pro Arg Phe Leu Ser Phe Lys Asn Glu Leu Ser Arg Tyr Leu Thr Asp
    50                  55                  60

His Phe Pro Ala Asn Val Asp Glu Tyr Gly Arg Val Tyr Gly Asn Gly
65                  70                  75                  80

Val Arg Thr Asn Phe Phe Gly Met Arg His Met Asn Gly Phe Pro Met
                85                  90                  95

Ile Pro Ala Thr Trp Pro Leu Ala Ser Asn Leu Lys Lys Arg Ala Asp
            100                 105                 110

Ala Asp Leu Ala Asp Gly Pro Val Ser Glu Arg Asp Asn Leu Leu Phe
        115                 120                 125

Arg Ala Ala Val Arg Leu Met Phe Ser Asp Leu Glu Pro Val Pro Leu
    130                 135                 140

Lys Ile Arg Lys Gly Ser Ser Thr Cys Ile Pro Tyr Phe Ser Asn Asp
145                 150                 155                 160

Met Gly Thr Lys Ile Glu Ile Ala Glu Arg Ala Leu Glu Lys Ala Glu
                165                 170                 175

Glu Ala Gly Asn Leu Met Leu Gln Gly Lys Phe Asp Asp Ala Tyr Gln
            180                 185                 190

Leu His Gln Met Gly Gly Ala Tyr Tyr Val Val Tyr Arg Ala Gln Ser
        195                 200                 205

Thr Asp Ala Ile Thr Leu Asp Pro Lys Thr Gly Lys Phe Val Ser Lys
    210                 215                 220
```

```
Asp Arg Met Val Ala Asp Phe Glu Tyr Ala Val Thr Gly Gly Glu Gln
225                 230                 235                 240

Gly Ser Leu Phe Ala Ala Ser Lys Asp Ala Ser Arg Leu Lys Glu Gln
                245                 250                 255

Tyr Gly Ile Asp Val Pro Asp Gly Phe Phe Cys Glu Arg Arg Arg Thr
            260                 265                 270

Ala Met Gly Gly Pro Phe Ala Leu Asn Ala Pro Ile Met Ala Val Ala
        275                 280                 285

Gln Pro Val Arg Asn Lys Ile Tyr Ser Lys Tyr Ala Tyr Thr Phe His
    290                 295                 300

His Thr Thr Arg Leu Asn Lys Glu Glu Lys Val Lys Glu Trp Ser Leu
305                 310                 315                 320

Cys Val Ala Thr Asp Val Ser Asp His Asp Thr Phe Trp Pro Gly Trp
                325                 330                 335

Leu Arg Asp Leu Ile Cys Asp Glu Leu Leu Asn Met Gly Tyr Ala Pro
            340                 345                 350

Trp Trp Val Lys Leu Phe Glu Thr Ser Leu Lys Leu Pro Val Tyr Val
        355                 360                 365

Gly Ala Pro Ala Pro Glu Gln Gly His Thr Leu Leu Gly Asp Pro Ser
    370                 375                 380

Asn Pro Asp Leu Glu Val Gly Leu Ser Ser Gly Gln Gly Ala Thr Asp
385                 390                 395                 400

Leu Met Gly Thr Leu Leu Met Ser Ile Thr Tyr Leu Val Met Gln Leu
                405                 410                 415

Asp His Thr Ala Pro His Leu Asn Ser Arg Ile Lys Asp Met Pro Ser
            420                 425                 430

Ala Cys Arg Phe Leu Asp Ser Tyr Trp Gln Gly His Glu Glu Ile Arg
        435                 440                 445

Gln Ile Ser Lys Ser Asp Asp Ala Met Leu Gly Trp Thr Lys Gly Arg
    450                 455                 460

Ala Leu Val Gly Gly His Arg Leu Phe Glu Met Leu Lys Glu Gly Lys
465                 470                 475                 480

Val Asn Pro Ser Pro Tyr Met Lys Ile Ser Tyr Glu His Gly Gly Ala
                485                 490                 495

Phe Leu Gly Asp Ile Leu Leu Tyr Asp Ser Arg Arg Glu Pro Gly Ser
            500                 505                 510

Ala Ile Phe Val Gly Asn Ile Asn Ser Met Leu Asn Asn Gln Phe Ser
        515                 520                 525

Pro Glu Tyr Gly Val Gln Ser Gly Val Arg Asp Arg Ser Lys Arg Lys
    530                 535                 540
```

```
Arg Pro Phe Pro Gly Leu Ala Trp Ala Ser Met Lys Asp Thr Tyr Gly
545                 550                 555                 560

Ala Cys Pro Ile Tyr Ser Asp Val Leu Glu Ala Ile Glu Arg Cys Trp
                565                 570                 575

Trp Asn Ala Phe Gly Glu Ser Tyr Arg Ala Tyr Arg Glu Asp Met Leu
            580                 585                 590

Lys Arg Asp Thr Leu Glu Leu Ser Arg Tyr Val Ala Ser Met Ala Arg
        595                 600                 605

Gln Ala Gly Leu Ala Glu Leu Thr Pro Ile Asp Leu Glu Val Leu Ala
    610                 615                 620

Asp Pro Asn Lys Leu Gln Tyr Lys Trp Thr Glu Ala Asp Val Ser Ala
625                 630                 635                 640

Asn Ile His Glu Val Leu Met His Gly Val Ser Val Glu Lys Thr Glu
                645                 650                 655

Arg Phe Leu Arg Ser Val Met Pro Arg
            660                 665
```

**Description**

## FIELD OF THE INVENTION

This invention relates to a novel polymerase protein capable of RNA synthesis in the presence of different RNA and DNA templates. The invention also relates to a method and a kit for the RNA synthesis by contacting the said polymerase protein with different RNA and DNA templates under appropriate conditions. This invention relates also to methods for stabilizing and sequencing nucleic acids.

## BACKGROUND OF THE INVENTION

Double-stranded RNA viruses are known to infect different hosts from prokaryotes to higher eukaryotes. Some of these viruses cause severe infectious diseases affecting humans and economically important animals and plants (Fields and Knipe, 1990). In spite of notable variations in structural organization and host specificity, practically all dsRNA viruses share a common replication strategy. Upon entry, the virion in most cases is converted into a core particle that functions as a transcriptase producing positive-sense single-stranded RNAs using the genomic dsRNAs as templates. The ssRNAs formed in the viral core are extruded into the cytoplasm where they serve as the messengers directing protein synthesis. The same ssRNAs are also fully active as templates for the synthesis of complementary minus-strands (replication). This process occurs inside the newly assembled core particles and is driven by the viral polymerase. After replication, the minus-strand RNA replica remains associated with the plus-strand template reconstituting the genomic dsRNA. The core particles bearing the dsRNA can either support additional rounds of transcription or alternatively undergo further maturation to form infectious progeny particles. Both replication and transcription of dsRNA viruses thus depend on the virus-encoded polymerase activities and occur in the interior of a large protein complex. Among several proteins building up the polymerase complex of any dsRNA virus, only one polypeptide species has been predicted theoretically to contain several characteristic sequence motifs conserved across RNA polymerases (Koonin *et al.,* 1989; Bruenn, 1991; Bruenn 1993). However, no direct biochemical evidence on the polymerase activity of such proteins has been available thus far.

Several experimental systems have so far been developed to shed light on the molecular principles that govern the RNA metabolism within the polymerase complexes of dsRNA viruses. The first of those systems was *in vitro* transcription based on purified intact viruses or core particles derived from virus preparations and thus already containing dsRNA templates. Such systems have been reported for reovirus (Joklik, 1974), bacteriophage ϕ6 (Van Etten *et al.,* 1973; Partridge *et al.,* 1979), infectious pancreatic necrosis virus (Cohen, 1975), yeast virus-like particles (Herring and Bevan, 1977) and many others. These approaches have given detailed information on the mechanisms and regulation of ssRNA synthesis. However, the particle-based transcription did not allow one to address questions on the replication.

This was approached using isolated virus intermediates containing packaged ssRNA (see for example Fujimura *et al.,* 1986) and empty polymerase particles. In the case of phage ϕ6, empty recombinant polymerase complex particles (PC) were found to be active in the RNA packaging, replication and transcription *in vitro* (Gottlieb *et al.,* 1990; Olkkonen et al, 1990; Van Dijk *et al., 1995*). Two other systems, the yeast virus-like particles (VLPs) and rotavirus open-core particles, were demonstrated to support replication of virus-specific exogenous ssRNA templates (Fujimura and Wickner, 1988; Chen *et al.,* 1994).

Bacteriophage ϕ6 is a complex dsRNA virus of *Pseudomonas syringae* (Vidaver *et al.,* 1973). The ϕ6 genome consists of three dsRNA segments: large (L), medium (M) and small (S) (Semancik et al, 1973; Van Etten *et al.,* 1974). For purposes of the present invention, the plus-sense strands of the ϕ6 RNA segments will be referred to as $1^+$, $m^+$, $s^+$; and the minus-sense strands will be designated $1^-$, $m^-$, $s^-$, correspondingly. The entire polymerase complex of ϕ6 phage is composed of four protein species P1, P2, P4 and P7, all encoded on the L segment (Mindich *et al.,* 1988). P1 is the major structural protein assembled into a dodecahedral shell with the rest of the protein subunits most probably being located at the 5-fold symmetry positions (Butcher *et al.,* 1997; de Haas *et al.,* 1999). Studies on individual recombinant proteins and genetically engineered incomplete PC particles have allowed one to understand the functions of P4 and P7. P4 is a hexameric NTPase responsible for the plus-strand RNA packaging (Gottlieb *et al.,* 1992a; Paatero *et al.,* 1995; Frilander and Bamford, 1995; Juuti *et al.,* 1998; Paatero *et al*., 1998), while P7 serves as a protein cofactor necessary for the efficient packaging reaction (Juuti and Bamford, 1995, 1997). P2, thus far the least studied PC protein, has been identified as a putative polymerase subunit using the computer analysis of the protein sequence (Koonin *et al.,* 1989; Bruenn, 1991). This conclusion was further supported with the biochemical studies on different protein-deficient PC particles (Gottlieb *et al.,* 1990; Casini *et al*., 1994; Juuti and Bamford, 1995).

Can the putative polymerase of a dsRNA virus catalyze template-dependent RNA synthesis alone or is the synthesizing activity strictly associated with the particle-bound polymerase protein? Until recently, this question has remained unanswered. Neither protein P2 from the ϕ6 phage nor the analogous putative polymerases from other dsRNA viruses (except rotavirus polymerase) have been thus far obtained in an isolated form. This may have been due to the fact that the putative polymerase represent only a minor component of the polymerase complex thus discouraging any attempts to purify the protein directly from this complex. In addition, no gene expression system has been known that would allow one to produce individual soluble polymerase protein. For two putative polymerases, those from the bluetongue virus and the infectious bursal disease virus, some polymerase activity has been reported to be associated with the crude extracts of the cells producing the corresponding recombinant proteins (Urakawa *et al.,* 1989; Macreadie and Azad, 1993). However, these reports have not provided any evidence for direct association of the observed polymerase activities with the proteins of interest. On the other hand, the rotavirus putative polymerase VP1 has been shown to possess some relevant partial activities. The enzyme was found to bind a nucleotide analog (Valenzuela *et al.,* 1991) and the viral ssRNA (Patton, 1996). The isolated protein, however, failed to replicate RNA substrates unless supplemented with at least one additional protein VP2 (major structural protein) (Zeng *et al.,* 1996; Patton *et al.,* 1997).

In the present invention a polymerase is shown to be capable of RNA synthesis in *vitro* not assisted with any other proteins. As it will become evident from the following description, the polymerase of this invention is relatively unspecific to the template it uses for the RNA polymerization. On contrary, template specificity of the RNA-dependent polymerases of the prior art was generally rather strict. For example, RNA polymerase of bacteriophage Qβ replicates effectively very limited set of templates unless RNA-primer is annealed to the target RNA (U.S. Patent No. 5631129 and references therein). A specific 3'-terminal tRNA-like structure is essential for RNA replication with brome mosaic virus RNA polymerase (Dreher and Hall, 1988). Analogously, virus-specific elements are necessary for the RNA synthesis catalyzed by the polymerase from influenza virus (U.S. Patent No. 5854037). Finally, rotavirus open core particles have been shown to replicate only homologous ssRNAs (Chen et al., 1994; and U.S. Patent No. 5614403).

## SUMMARY OF THE INVENTION

The present disclosure concerns a novel, unspecific polymerase protein capable of primer-independent RNA synthesis in the presence of a variety of RNA and DNA templates. The inventors believe this to be the first report of isolating an RNA polymerase that is capable of effective primer-independent replication *in vitro* of a broad range of both virus-specific and heterologous ssRNA templates, to produce corresponding dsRNA products. In a preferred embodiment, the polymerase originates from a double-stranded RNA virus or from a cell containing a nucleic acid that encodes the polymerase of a double-stranded RNA virus. The characteristics of the polymerase of the invention make it particularly suitable for (1) amplification of RNA *in vitro*, (2) incorporation of easily detectable nucleotide analogs in a synthesis product, (3) RNA synthesis to produce very long dsRNAs, (4) stabilization of single- stranded nucleic acids, and (5) sequencing of polynucleotides.

The polymerase protein of this invention originates preferably from *Cystoviridae, Reoviridae, Birnaviridae* or *Totiviridae* viruses, specifically from ϕ6-related bacteriophages from the family *of Cystoviridae*, such as from ϕ6, ϕ7, ϕ8, ϕ9, ϕ10, ϕ11, ϕ12, ϕ13, or ϕ14 (Mindich et al., 1999).

The most preferred embodiment of this art deals with P2 polymerase of double-stranded RNA bacteriophage ϕ6. More specifically, P2 polymerase was isolated from a bacterial strain containing DNA encoding said protein. The preparation of isolated P2 polymerase was demonstrated in an *in vitro* enzymatic assay to act as a template-dependent RNA polymerase. It is found that P2 polymerase has low template specificity being able to catalyze RNA synthesis in the presence of ssRNA, dsRNA, ssDNA and dsDNA substrates, preferably in a linear form. The P2 polymerase is processive, has very high RNA-polymerization rate and does not require primer for the initiation of RNA synthesis, although it is also able to initiate RNA synthesis in the presence of a primer. This invention also relates to genetically modified forms of a P2 polymerase or other altered forms that are altered due to naturally occurring changes in the genetic code.

To the inventors' knowledge, this is the first known report of the isolation of an RNA polymerase capable of effective primer-independent replication *in vitro* of a broad range of both virus-specific and heterologous ssRNA templates to produce corresponding dsRNA products. Template specificity of other known RNA-dependent RNA polymerases is generally rather strict as described here earlier. The polymerase of this invention represents a new type of enzyme, which can be used in molecular biology as a general tool for producing dsRNA from virtually any given ssRNA template. Recently, dsRNA has become the subject of considerable interest as it has been shown to trigger a number of very important processes in different organisms (for review see Sharp, 1999).

The capability of the polymerase of this invention to utilize different dsRNA templates for the RNA synthesis *in vitro* is also a novel feature, which has not been known for any of the studied polymerases. Even though yeast virus-like particles have been previously reported to catalyze dsRNA-dependent RNA synthesis, the only template used for the RNA transcription in this system was a virus-specific dsRNA (Fujimura and Wickner, 1989). In this invention, term "RNA transcription" refers to the RNA synthesis on dsRNA templates. In the RNA transcription catalysed by P2 polymerase, newly synthesized RNA forms duplex with the template strand of the dsRNA template and displaces the old non-template strand. This type of reaction thus can be used to label a dsRNA substrate with radioactive or chemically modified nucleotides incorporated into resultant dsRNA product during incubation with the polymerase. Alternatively, the reaction can be used to recover ssRNA displaced from the substrate dsRNA.

The capability of the polymerase protein of the present invention to convert ssRNA to dsRNA and to transcribe dsRNA by the strand-displacement mechanism suggests the use of the enzymes to amplify RNA *in vitro.* Unlike conventional polymerases, the P2 protein does not require a primer to synthesize a complementary product of a single-stranded RNA template. Accordingly, the P2 polymerase is uniquely suited to amplify RNA substrates. These characteristics make the polymerase of the invention particularly useful in the context of detecting infection. A diagnostic method in this regard comprises amplifying the RNA sample and, optionally, incorporating easily detectable nucleotide analogs into the amplification product as well as identifying the RNA species by direct sequencing.

It is also highly advantageous that the polymerase of this invention is capable of RNA synthesis in the presence of DNA substrates. This feature allows production of desired DNA-RNA heteroduplexes suitable for both biological and physico-chemical studies. It also allows RNA synthesis from DNA templates in the presence of radioactively labeled or chemically modified nucleotides to yield DNA-RNA heteroduplexes radioactively labeled or containing chemically modified nucleotides, respectively.

The invention contemplates a method for *in vitro* RNA synthesis that employs polymerases of the invention. The method comprises: (a) providing a nucleic acid substrate which may belong to either ssRNA, or dsRNA, or ssDNA, or dsDNA as will be specified in the detailed description below; (b) contacting said substrate with a polymerase protein under conditions sufficient for the RNA synthesis; and (c) recovering the newly formed nucleic acids from the reaction mixture. This method for preparing dsRNA advantageously can be used to produce very long double-stranded RNAs, up to at least 13,500 bp, in contrast to the methods relying on RNA-RNA hybridization. Extant techniques described, for example, in U.S. Patent No. 5795715 typically produce dsRNAs of less then 1000 bp in length.

The polymerase protein of this invention can be used in methods for stabilizing nucleic acids. Single-stranded nucleic acids are known to be easily degradable by nucleases. By converting single-stranded nucleic acids to double-stranded nucleic acids and contacting the reaction mixture with a preparation containing nuclease or nucleases, it is possible to recover double-stranded nucleic acids which has increased stability compared to single-stranded nucleic acids.

The present invention also provides a kit for the template-dependent RNA-synthesis *in vitro,* as will be described below.

The present invention relates also to a method for producing dsRNA from dsDNA. The method comprises:

(a) providing ssRNA substrate by transcribing a DNA template with a DNA-dependent RNA polymerase; and
(b) converting ssRNA substrate to dsRNA with the protein of the invention, wherein steps (a) and (b) are preferably carried out at the same time or sequentially in the same reaction vessel.

The present invention is also directed to methods for determination of nucleotide base sequence of a nucleic acid molecule using the polymerase protein of this invention. This opens up the possibility to direct sequencing of nucleic acids without primer. A kit specifically for sequencing nucleic acid molecules is also disclosed.

Other features, aspects and advantages of the present invention will become apparent from the following description and appended claims.

## BRIEF DESCRIPTION OF THE FIGURES

The foregoing text, as well as the following detailed description of the present invention, will be better understood when read in conjunction with the appended figures in which:

Fig. 1 shows the purification of the recombinant P2 produced in *E. coli* cells. P2 expression was performed at 15°C for 18 h as described in Example 1. **(A)** SDS-PAGE gel stained with Coomassie Blue G-250. Lanes: protein composition of bacterial cells BL21 (DE3/pEM2) before (1) and after (2) induction of P2 synthesis with IPTG; cleared cell lysate (3); samples after successive purification on Cibacron Blue agarose (4), heparin agarose (5), and the Resource Q column (6). Proteins of the wild-type $\phi$6 are marked on the right. **(B)** Immunoblot analysis of the same protein samples using antibodies raised against the entire $\phi$6 polymerase complex (proteins P1, P2, P4, and P7). Lane designation is as in **(A).**

Fig. 2 depicts recombinant P2-catalyzed RNA synthesis *in vitro* in the presence of a ssRNA template. Agarose gel analysis of aliquots from the standard 10 $\mu$l polymerase assay mixtures containing (except lane 8) the synthetic single-stranded positive-sense m-segment of the $\phi$6 phage ($m^+$ RNA; 100 $\mu$g/ml). The critical additives are indicated below the panels. P2 refers to the purified P2 protein (lane 6 in Fig. 1). P2-CBA is partially purified P2 after the Cibacron Blue agarose column (Fig. 1, lane 4) and mock-CBA is the analogously prepared protein fraction derived from IPTG induced BL21(DE3) cells containing pET32b(+) plasmid. The position of the labelled $\phi$6 segments produced in the nucleocapsid transcription (N) is shown on the left. Double-stranded segments are marked with capital letters (L, M and S), and the plus-sense single-stranded segments are shown in lowercase ($1^+$, $m^+$ and $s^+$). (**A**) EtBr stained gel; (B) autoradiogram of the same gel.

Fig. 3 depicts that the product of the RNA synthesis is dsRNA formed by the template and the complementary newly produced strand. Products of the $m^+$ RNA replication assay analyzed in a strand-separating gel. Lanes marked with p contained P2 protein in the assay (same conditions as in the lane 4 of Fig. 2); those marked with b were supplemented with an equal amount of the P2 control buffer (same as in lane 2 of Fig. 2). Lanes marked with N contain labelled ϕ6 segments produced in the nucleocapsid transcription. Double-stranded RNA segments were heat-denatured (boiled) to yield individual plus ($1^+$, $m^+$ and $s^+$) and minus ($1^-$, $m^-$ and $s^-$) RNAs. No strand separation occurred if the boiling step was omitted (not boiled). Panel **(A)** is EtBr stained gel; **(B)** is the autoradiogram of the same gel. **(C)** RNase protection assay. Reaction products purified from the P2 (p) or the control (b) replication mixtures containing [$\alpha^{32}$P]UMP labeled $m^+$ RNA template and no labeled nucleotide triphosphates were incubated with (+RNase) or without (-RNase) addition of RNase I and analyzed in the standard agarose gel.

Fig. 4 depicts that the replicase activity is associated with the monomer of P2. Purified P2 was analyzed in the Superdex 75 gel-filtration column and the replicase activity was determined in the collected fractions. Peak of the replicase activity coincides with the P2 protein peak. **(A)** Absorbance (280 nm) profile of the eluate from the column. Arrows indicate the P2 injection time (inject) and position of the molecular mass standards: BD, Blue Dextran (2000 kDa); βAm, β-amylase (200 kDa); IgG, mouse immunoglobulin G (150 kDa); BSA, bovine serum albumin (67 kDa); OA, ovalbumin (45 kDa); STI, soybean trypsin inhibitor (20.1 kDa); αLA, α-lactalbumin (14.2 kDa). Inset, SDS-PAGE analysis of the protein content in fractions 9 to 22. **(B)** Autoradiogram of the agarose gel showing replicase activity in fractions 1 to 29. Lane N is as defined in Fig. 2.

Fig. 5 depicts that P2 non-specifically replicates ssRNA substrates. **(A)** EtBr stained gel showing replication products of the reactions containing the purified P2 protein (p) or the control buffer (b). Single-stranded RNA substrates used to program reactions were as follows. 1, $1^+$ RNA (synthetic positive-sense large segment of the ϕ6 phage produced with T7 transcription of pLM687 treated with *XbaI* and mung bean nuclease, MBN); 2, $m^+$ RNA (medium segment, same as in Fig. 2); 3, $s^+$ RNA (small segment; T7 transcript of pLM659 treated with *Xba*I and MBN); 4, shortened $s^+$ RNA (T7 transcript of pLM659 cut with *Eco*47111); 5, extended $s^+$ RNA (T7 transcript of pLM659 cut with *Sma*I); 6, extended $s^+$ RNA (T7 transcript of pLM659 cut with *Eco*RI)*;* 7, 13.5 kb long RNA containing fused $s^+$, $m^+$ and $1^+$ segments (T7 transcript of pLM1809 treated with *Xba*I and MBN); 8, mixture of natural $s^+$, $m^+$ and $1^+$ segments purified from the ϕ6 nucleocapsid-directed transcription; 9, RNA mixture produced by T7 transcription of the entire DNA of bacteriophage T7; 10, firefly luciferase mRNA (SP6 transcript of pGEMluc cut with *Stu*I*);* 11, genome RNA of the coliphage MS2 (Boehringer); 12, mixture of bluetongue virus, strain 1 (BTV1) ssRNA segments LiCl precipitated from the BTV1 nucleocapsid transcription. ϕ6 segments from nucleocapsid transcription (N) are marked on the left. Positions of the ten genomic dsRNA segments phenol extracted from the virions of BTV1 are shown on the right (B1-B10). (**B**) Autoradiogram of the same gel.

Fig. 6 depicts the time course of P2-directed replication. **(A)** The 100 μl replication mixture programmed with the three natural positive-sense segments was incubated at 28°C in the presence of the P2 protein. 5 μl aliquots, sampled at the time points indicated, were analyzed in the standard agarose gel and autoradiographed. Lane N is as in Fig. 2. **(B, C** and **D)** The phosphoimager (Fuji BAS 1500) analysis of the time-dependent accumulation of replication products L, M and S, respectively. The graphs are normalized so that the highest observed value within each panel is set to 100%. Insets in **B, C** and **D** show the first 300 s of the time courses. Lines extrapolate linear parts of the plots to the time axis. $\tau_L$, $\tau_M$ and $\tau_S$ indicate the duration of the lag phases prior to the appearance of relevant full-length dsRNA segments.

Fig. 7 depicts that P2 initiates replication from the very 3'-terminal nucleotide of the ssRNA template. RNA products of the replication reactions programmed with the mixture of natural ssRNA segments $s^+$, $m^+$ and $1^+$ and containing P2 protein (p) or buffer (b) were assayed in the primer extension experiment with a labeled primer complementary to the minus-strand (s) of the small ϕ6 segment. As a control, primer extension was also done on the heat denatured dsRNA genome (d) extracted from wild type ϕ6. Dideoxynucleotide termination sequencing lanes (A, C, G and T) are boxed. They were produced with the same primer and T7 Sequenase 2.0 (Amersham) using plasmid pLM659 containing cloned cDNA of the $s^+$ segment. Sequence reading is shown on the left. The 3'-terminal "T" of $s^+$ is marked with the arrow and the unique restriction sites mentioned in Fig. 5 are underlined.

Fig. 8 depicts that P2 polymerase catalyzes RNA synthesis (transcription) in the presence of dsRNA templates. EtBr stained gel **(A)** and autoradiogram of the same gel (B) show products of the reactions containing purified P2 protein (p) or the control buffer (b). Double-stranded RNA substrates were as follows. ϕ6, mixture of genomic dsRNA segments extracted from bacteriophage ϕ6; L-A, genomic dsRNA of *Saccharomyces cerevisiae* virus L-A; BTV1, mixture of genomic dsRNA segments of bluetongue virus, strain 1. Positions of L, M and S segments of ϕ6 are shown on the left, those of the ten BTVI segments (B1-B10) are shown on the right.

Fig. 9 depicts that P2-catalyzed transcription of ϕ6-specific dsRNA substrates results in the synthesis of predominantly plus-sense RNA strands. **(A)** Two ssRNA and two dsRNA substrates were incubated at 28°C in separate reaction mixtures containing purified P2 (p) or control buffer (b). Aliquots were taken out of the mixtures at 1h point and analyzed in a standard agarose gel. Single-stranded RNAs were as follows: ϕ6ss, mixture of natural $s^+$, $m^+$ and $1^+$ segments purified from the ϕ6 nucleocapsid-directed transcription; $m^+$, $m^+$ RNA. Double-stranded RNAs: ϕ6ds, mixture of the three genomic segments extracted from bacteriophage ϕ6; M, synthetic M segment prepared by replication of ssRNA $m^+$ with P2 and subsequent purification of the newly formed dsRNA using a standard agarose gel-electrophoresis. Lane N is as defined in Fig. 2. **(B)** Strand-separating electrophoresis of the reaction products prepared in the presence of P2 as described in **(A)** and heat-treated before the gel-analysis.

Fig. 10 depicts that P2 polymerase catalyzes RNA synthesis in the presence of DNA templates. **(A)** ssDNA of M13mp10 cut with *Hin*f1 was incubated with (1) or without (2) P2 polymerase as described in Example 3. N is the marker lane as in Fig. 2. **(B)** dsDNAs of pUC18 cut with different restriction endonucleases: 1, *Hinc*II*;* 2, *Sma*I*;* 3, *Kpn*I; 4, *Pst*I; 5, *Sad*I; 6, *Bam*HI; 7, *Hind*III; 8, *Xba*I*,* were incubated with P2 polymerase and analyzed as described in Example 3. Lane 9 is the result of incubating pUC18 cut with *Xba*I in the reaction mixture without P2.

Fig. 11 demonstrates incorporation of nucleotide analogs into newly produced RNA. Standard P2 replication mixtures were supplemented with **(A)** 25 μM of Alexa Fluor ® 488-5-UTP; **(B)** 25 μM of coumarin-5-CTP; or **(C)** 100 μM biotin-11-CTP. For each of these nucleotide analogs, three separate reactions were carried out containing: **(P)** P2 polymerase (40 μg/ml) and no RNA; **(R)** $ms^+$ ssRNA template (75 μg/ml; Makeyev and Bamford (2000) EMBO J., 19, 6275-6284) and no polymerase; **(PR)** both P2 polymerase and the ssRNA template. After 1 h incubation at 30 °C, reactions were passed through gel-filtration columns the flow-through fractions (containing the RNA reaction products) being used for further analysis. Fluorescence emission spectra of the purified fractions recorded at the fixed excitation wavelengths: 490 nm **(A);** 402 nm **(B). (C)** Biotin-streptavidin dot-blot assay. Briefly, aliquots from the flow-through fractions were spotted at the Hybond N + nylon membrane (Amersham) and were stained with streptavidin-HRP conjugate (NEN), which was followed by the ECL detection step (kit from Biological Industries, Israel).

## DETAILED DESCRIPTION OF THE INVENTION

A polymerase protein of the present invention originates from a dsRNA virus or has the amino acid sequence of such a viral polymerase. A polymerase of the invention catalyzes RNA synthesis using ssRNA, dsRNA, ssDNA, or dsDNA templates. A key aspect of the invention is a method for purifying a polymerase from a dsRNA virus. A preferred polymerase of the invention, the P2 polymerase, is processive, has very high RNA-polymerization rate, and does not require primer for the initiation of RNA synthesis, although it also is able to initiate RNA synthesis in the presence of a primer. As noted above, primer-independent synthesis is especially useful in amplifying RNA for quantitation of RNA species in the sample and their identification by direct sequencing. This methodology is especially useful in detecting pathogenic parasites and differences in gene expression levels associated with diseases.

### Polymerases of this invention

A novel type of template-dependent RNA polymerases has very low template specificity and is able to catalyze RNA synthesis in the presence of different nucleic acid substrates. The RNA polymerase is variously referred to as "polymerase of a dsRNA virus", "dsRNA virus polymerase", "polymerase protein" or "polymerase". This invention provides the first direct evidence that the isolated polymerase originating from a dsRNA virus alone is capable of RNA synthesis *in vitro* when contacted with a ssRNA, dsRNA, ssDNA, or dsDNA substrate under suitable conditions.

Preferably, the RNA polymerase of this invention may originate from any dsRNA virus (e.g. from *Cystoviridae, Reoviridae, Birnaviridae or Totiviridae).* ϕ6-related bacteriophages from the family of *Cystoviridae* (e.g. ϕ6, ϕ7, ϕ8, ϕ9, ϕ10, ϕ11, ϕ12, ϕ13 or ϕ14) are expected to be the most preferable origin of the polymerase of this invention.

Identical or substantially similar polymerases may be prepared by isolating a nucleic acid with a sequence encoding an identical or substantially similar protein, expressing said protein under suitable regulatory regions in a chosen host and isolating the protein. A nucleic acid with a sequence encoding such a protein is preferably isolated from dsRNA viruses or it may be synthetic or partially synthetic.

In a preferred embodiment of the invention the *in vitro* system for the RNA synthesis is based on the purified recombinant protein P2 of the dsRNA bacteriophage ϕ6. The P2 protein of the dsRNA bacteriophage ϕ6 also is referred variously herein as "RNA polymerase P2", "P2 RNA polymerase", "P2 polymerase", "P2 protein", or "P2".

The present invention relates furthermore to proteins, which are encoded by a nucleic acid sequence selected from the group comprising:

(a) a nucleic acid sequence having at least a partial nucleic acid sequence of SEQ ID NO:1;
(b) a nucleic acid sequence encoding a polypeptide having at least a partial amino acid sequence of SEQ ID NO:8;
(c) a nucleic acid sequence which differs from the nucleic acid sequence of (a) or (b) due to degeneracy of the genetic code;

(d) a nucleic acid sequence hybridizing to the nucleic acid sequence of (a), (b) and/or (c); and

(e) a nucleic acid sequence encoding an amino acid sequence which shows at least 20 % identity, preferably at least 50% identity to a sequence contained in (b).

A "partial nucleic acid sequence" means a continuous RNA or DNA sequence lacking at least one nucleotide from one or the other end of SEQ ID NO:1, the partial sequence being still capable of regulating the expression of a protein having similar biological activity as the protein P2.

A "partial amino acid sequence" means a continuous amino acid sequence lacking at least one amino acid from one or the other end of SEQ ID NO:8 having still similar biological activity as the protein P2. In preferred embodiments, the partial amino acid sequence lacks 10, 30, or 50 amino acids from the N-terminal and/or C-terminal end of the polypeptide.

The present invention relates also to nucleic acid sequences, which differ from SEQ ID NO:1 due to degeneracy of the genetic code.

The present invention relates furthermore to nucleic acid sequences, which hybridize to the SEQ ID NO:1 under conventional hybridization conditions, preferably under stringent conditions such as described by Sambrook *et al.*, 1989. High stringency hybridization may be between about 65 °C and 70 °C in a solution of 6X SSC, 0.5 % SDS, 5X Denhardt's solution and 100μg of non-specific carrier DNA. The preferred probe is 100 bases selected from contiguous bases of the polynucleotide sequence set forth in SEQ ID NO:1. Excess probe is removed by washing in a solution having the equivalent ionic strength of less than about 0.2X to 0.1X SSC. A typical high stringency wash is twice for 30 minutes at 55 °C. and three times for 15 minutes at 60 °C.

These nucleic acid sequences that hybridize to the nucleic acid sequences of the present invention can in principle be derived from any organism possessing such nucleic acid sequences. Preferably, they are derived from dsRNA viruses. Nucleic acid sequences hybridizing to the nucleic acid sequences of the present invention can be isolated, e.g., from genomic libraries of various organisms.

Such nucleic acid sequences can be identified and isolated by using the nucleic acid sequences of the present invention or fragments of these sequences or the reverse complements of these molecules, e.g. by hybridization according to standard techniques (see Sambrook *et al.,* 1989).

As hybridization probe can be used nucleic acid molecules that have exactly or substantially the same nucleotide sequence as SEQ ID NO:1 or fragments of said sequence. Preferably is used the entire nucleotide sequence SEQ ID NO:1. The fragments used as hybridization probes can also be synthetic fragments obtained by conventional synthesis techniques, the sequence of which is substantially identical to that of the nucleic acid sequences of the invention. Once genes hybridizing to the nucleic acid sequences of the invention have been identified and isolated it is necessary to determine the sequence and to analyze the properties of the proteins coded for by said sequence.

The term "hybridizing nucleic acid sequence" includes fragments, derivatives and allelic variants of SEQ ID NO:1 encoding an identical or substantially similar protein or a biologically active fragment thereof. Fragments are understood to be parts of nucleic acid sequences long enough to code for the described protein (or substantially similar protein) or a biologically active fragment thereof. The term "derivative" means in this context that the nucleotide sequences of these molecules differ from the sequences of the above-described nucleic acid molecules in one or more positions and are highly homologous to said sequence.

" % Identity" means here percentage of identical amino acids being present at corresponding positions when two amino acid sequences are aligned to give the maximal amount of identical nucleotides or amino acids at corresponding positions. This invention relates to proteins, the amino acid sequence of which has at least 20%, preferably at least 50%, more preferably at least 80%, even more preferably at least 85%, still more preferably at least 90%, and most preferably at least 95% identity at the amino acid level to the specific amino acid sequence of SEQ ID NO:8.

Protein engineering can be used to construct modified polymerases possessing improved properties. Such modifications may, for example, include mutating amino acid sequence of P2 polymerase or a protein with substantially similar properties in order to make said polymerase: 1) less template-specific; 2) more processive; or 3) more efficient in catalysis of RNA synthesis on double-stranded nucleic acids templates, than the enzyme available at the moment. Such modification may include also optimizing the enzyme for primer extension, sequencing or for amplification of nucleic acids.

**Production of the polymerase protein of this invention**

This invention provides a method of expression and purification of the protein of this invention, preferably a dsRNA virus polymerase protein. The method comprises (a) culturing a cell containing nucleic acid with a sequence encoding a polymerase protein of this invention to express said protein from said nucleic acid with a sequence; (b) recovering the protein from the host or from the culture medium; and (c) purifying said protein. The nucleic acid sequences of this invention may be operably linked to the regulatory elements in an expression vector, which is introduced into a chosen host cell to produce the protein under the control of the sequences. As a specific embodiment, expression and purification of P2 RNA polymerase of bacteriophage $\phi$6 is dealt with in Example 1.

Expression of the polymerase of this art may be achieved in any suitable host cells (e.g., animal, plant, fungal or bacterial cells). In the currently prefered embodiment of this invention, expression host is bacterium *Escherichia coli.*

The protein is preferably isolated and purified by the steps, comprising:

(a) disrupting the host cells in a buffer to obtain a cell lyzate;
(b) clarifying said lyzate by centrifugation;
(c) purifying the protein using at least one step, more preferably two steps of affinity chromatography;
(d) further purifying the protein using at least one step of ion exchange chromatography to obtain a fraction that is essentially free of nuclease and protease activities.

The purification method preferably comprises:

(i) purifying the protein on Blue Agarose;
(ii) further purifying the protein on Heparin Agarose; and
(iii) further purifying the protein on Resource Q to obtain a protein fraction essentially free of nucleases and proteases and containing at least 90 % , more preferably at least 95% of the polymerase protein.

"Essentially free of nucleases and proteases" means here that the purified protein preparation does not contain a detectable amount of nucleases and/or proteases.

**RNA synthesis on RNA substrate *in vitro***

The present invention relates to a method for producing RNA *in vitro,* comprising the steps of:

(a) providing ssRNA or dsRNA substrate;
(b) contacting said ssRNA or dsRNA substrate with the protein of the invention under conditions sufficient for RNA synthesis; and
(c) recovering the newly produced dsRNA species from the reaction mixture.

In accordance with a specific embodiment of the present art, the polymerase P2 was shown to initiate *de novo* and further catalyze synthesis of the full-length complementary strand on a ssRNA substrate yielding a dsRNA product of the appropriate size (Fig. 2, Fig. 3 and Fig. 5). The reaction based on purified P2 can therefore be considered the first *in vitro* model of bona fide replication established for $\phi$6, because the $\phi$6 procapsid-based system reported previously (Olkkonen et al, 1990; Gottlieb *et al.,* 1990) can not support replication unless RNA packaging is completed (Frilander *et al.,* 1992).

In a specific embodiment of this art (Fig. 2) the P2 replication mixture, in addition to P2 protein, contained single-stranded $m^+$ RNA substrate (positive-sense m segment of the $\phi$6 phage), four nucleotide triphosphates (NTPs) including [$\alpha^{32}$P]UTP and the same buffer as described for the RNA synthesis in the recombinant procapsid system (Van Dijk *et al.,* 1995). Analysis of the reaction products showed that the presence of P2 protein correlated with the appearance of a new RNA band migrating as double-stranded form (M) of the $m^+$ RNA substrate and visible both on ethidium bromide (EtBr) stained gel and the autoradiogram (Fig. 2). The band intensity was proportional to the amount of added P2 in the tested range (lanes 2-4). No band appeared if P2 was substituted with BSA or the reaction mixture lacked the RNA substrate (lanes 2 and 1).

Characterization of the P2 replication products in the RNase protection assay carried out as shown in Fig. 3C unequivocally proves that the newly produced RNA does have dsRNA nature. It is also evident from the strand-separating experiment (Fig. 3A and B) that in the presence of a plus-sense RNA segment (specifically, $m^+$ segment) P2 polymerase synthesizes complementary minus-sense RNA strand.

According to a specific embodiment of this invention, the mixture for the RNA synthesis contained 0.01 to 0.1 mg/ml of purified P2 (see Fig. 1, lane 6), 40 $\mu$lg/ml to 300 $\mu$g/ml of RNA substrate, 50 mM Tris HCl, pH 8.9, 80 mM ammonium acetate ($NH_4OAc$), 1 mM each of ATP and.GTP, 0.2 mM each of CTP and UTP, 6% (w/v) PEG4000, 5 mM $MgCl_{2,}$ 1 mM $MnCl_2$, 2 mM DTT, 0.1 mM EDTA, 0.2 mg/ml BSA, and 800 u/ml RNasin. The reaction mixture was incubated at 28°C for 1 h. Somewhat modified conditions have been also shown to support a detectable level of RNA synthesis. Specifically, in the case $m^+$ RNA template is used, these modified conditions may imply one or several changes selected from the group:

(1) a different final concentration of P2 protein in the reaction mixture (preferably 0.005 to 0.4 mg/ml),
(2) less purified P2 protein preparation in the reaction mixture (Fig 2, lane 3);
(3) a buffer with a different pH value (preferably pH 7.3 to 9.3);
(4) a different concentration of nucleoside triphosphates (preferably 0.2 to 3 mM of each NTP);
(5) a different concentration of PEG 4000 (preferably 0 to 9%);
(6) a different concentration of $MgCl_2$ (preferably 0 to 10 mM, more preferably 5 to 10 mM);
(7) a different concentration of $MnCl_2$ (preferably 0 to 3 mM)
(8) a different concentration of BSA (preferably 0 to 1 mg/ml);
(9) a different temperature of incubation (preferably 20 to 42°C).

It is most advantageous, however, to increase the final concentration of both ATP and GTP to at least 1 mM for the optimal RNA synthesis (compare for example lanes 4 and 6 in Fig. 2). It is also highly advantageous to include $Mn^{2+}$ ions into the reaction mixture, because addition of $Mn^{2+}$ was found to considerably enhance RNA synthesis (compare for example lanes 4 and 5 in Fig. 2). In addition, the inventors have found that addition of a non-ionic detergent, preferably Triton X-100 or Tween 20, preferably up to the final concentration of 0.01 to 0.5%, is also advantageous to the reaction efficiency. The stimulatory effects of purine nucleotide triphosphates and manganese on the RNA-dependent RNA synthesis have been reported for both ϕ6 polymerase complex (Van Dijk *et al.,* 1995) and some other viral polymerases (Blumenthal, 1980, and references therein).

In another embodiment of the invention, P2 polymerase was demonstrated to replicate several different ssRNA substrates both related to ϕ6 phage and heterologous. *First*, a set of various ϕ6-specific ssRNAs was tested in the P2 polymerase assay (Fig. 5, lanes 1-8). Exact copies of both large ($1^+$) and small ($s^+$) ssRNA segments of the ϕ6 phage gave rise to the labeled dsRNA products migrating in the gel at the positions of L and S, respectively. The replication efficiency of these two substrates was very close to that of the $m^+$ RNA (Fig. 5, lanes 1-3) also used as a substrate in the previously discussed embodiments. Comparable replication efficiency was found for the natural single-stranded segments isolated from the ϕ6 nucleocapsid transcription mixture (lane 8). Another tested substrate was the 13.5 kb long transcript consisting of fused $s^+$, $m^+$, and $1^+$ segments (Qiao *et al.,* 1997). The double-stranded product in this case migrated notably slower than L segment indicating complete or almost complete replication. It is worth noting that the 70-80 base long 3'-terminal part of all three ϕ6 segments is conserved and believed to form extensive secondary structure (Mindich *et al.,* 1994). All the RNA substrates mentioned above contained this feature and were replicable. It was thus interesting to study the possible effect of the 3'-proximal sequence on the RNA replicability *in vitro.* A truncated $s^+$ segment lacking 158 nucleotides at the 3' terminus was synthesized and used as a substrate in the replication reaction (lane 4). Surprisingly, no reduction in the product yield was observed. On the contrary, the replication efficiency was somewhat higher than that of the unmodified $s^+$. Even more efficient replication was detected for $s^+$ RNA extended with 13 extra nucleotides originating from the plasmid polylinker (lane 5). However addition of 31 polylinker nucleotides to the $s^+$ segment significantly reduced yield of the dsRNA product (lane 6). Thus we conclude that: 1) neither the conserved secondary structure nor the ϕ6 specific sequence at the very 3' terminus of an RNA substrate are critical for the P2-directed replication *in vitro;* however 2) replication efficiency does depend on the substrate 3' terminal sequence. *Second*, P2 polymerase was also demonstrated to effectively replicate a number of heterologous ssRNA templates, not related to any of the three ϕ6 RNA segments. All of the RNAs tested turned out to be suitable substrates for the replication reaction, though the yield of the produced dsRNA depended on the nature of the input template (Fig. 5, lanes 9-12). The mixture of T7 phage transcripts resulted in a very effective synthesis of several dsRNA species (lane 9). Effective templates were the firefly luciferase messenger RNA and the plus-sense transcripts of the bluetongue virus (lanes 10 and 12). Replication of genomic RNA of the coliphage MS2 was reproducibly inefficient leading to a barely visible dsRNA product in the original EtBr stained gel. Even in this case the product band was clearly detectable on the autoradiogram (lane 11). Additionally, some other RNAs, namely mRNAs encoding thioredoxin (T7 transcript of pET32b(+) cut with *Xho*I*),* green fluorescent protein (T7 transcript of pTU58 cut with EcoRI) and firefly luciferase fused with neomycin phosphotrasferase II (17 transcript of pTZluc(NPT2) cut with *Xho*I), and a mixture of 16S and 23S ribosomal RNAs of *E. coli* (Boehringer), were also replicable with the P2 protein (data not shown). It is also worth noting, that the enzyme showed high processivity being able to replicate RNA templates up to 13.5 kb in length (Fig. 5, lane 7) and probably even longer (Fig. 5, lane 9).

Complete and effective replication of different ssRNA templates suggests a general method for producing , dsRNA *in vitro* using the polymerase protein of this invention. This method appears to be particularly useful knowing the importance of dsRNA as a powerful regulator of many cellular processes (Sharp, 1999). For several decades dsRNA molecules have been known as potent inhibitors of translation in higher eukaryotes. Recently, dsRNA has been demonstrated to cause so-called RNA interference (RNAi) in some animals like insects, nematodes, trypanosomes and zebrafish. Additional information on the dsRNA-mediated biochemical pathways comes from plants, which have been shown to respond to dsRNA in the form of posttranscriptional gene silencing (PTGS). Notably, both RNAi and PTGS are sequence-specific mechanisms implying that expression of a target gene is inhibited with a dsRNA fragment having sequence homologous to the gene or to its part. Further examples of a dsRNA-dependent regulation are very likely to be discovered in the nearest future. In this respect, provided method for the synthesis of dsRNA with the predetermined sequence represents a genuine breakthrough in both research and possible applications of dsRNA-triggered mechanisms. In a preferred embodiment of the invention, polymerase used in the method of dsRNA production is the RNA polymerase P2 originating from bacteriophage $\phi$6. The ssRNA substrate for the method can be either produced *in vitro* or purified from cellular or viral sources. Conditions suitable for the ssRNA replication can be either as it was described in a specific embodiment of this art (see Example 2), or modified in a way not compromising production of a detectable amount of the dsRNA product. Depending on the user's needs and intentions, produced dsRNA can be used with or without further purification from the other components of the reaction mixture.

In a specific embodiment of the present art, P2 was also shown to use double-stranded RNAs, purified from $\phi$6 or unspecific dsRNA genomes of other viruses (L-A, BTV, CPV) as templates for the RNA synthesis (Fig. 8 and not shown). In this invention, the reaction of RNA synthesis in the presence of a dsRNA substrate is referred to as RNA-transcription. As a result of the RNA-transcription, newly synthesized RNA forms duplex with the template strand of the dsRNA template and displaces the old non-template strand. In the case of the $\phi$6-derived dsRNA substrate, P2 is shown to synthesize predominantly plus-strand RNA (Fig 9).

**RNA synthesis on DNA substrate**

This invention relates to a method for producing RNA *in vitro,* comprising the steps of:

(a) providing ssDNA or dsDNA substrate;
(b) contacting said ssDNA substrate with the protein of the invention under conditions sufficient for RNA synthesis; and
(c) recovering the newly produced nucleic acid species from the reaction mixture.

In addition to naturally occurring dsRNAs, P2 also catalyzes RNA-transcription on synthetic dsRNA templates (Fig 9A, $m^+$ prepared from ssRNAs using the method for dsRNA production claimed in this invention. Specifically, the following steps were used to prepare synthetic dsRNA substrate for the above experiment (see also Example 2 for details):

a) producing $m^+$ ssRNA using *in vitro* transcription with the T7 RNA polymerase of a linearized plasmid containing $m^+$ cDNA copy under the control of a T7 promoter;
b) replication of the $m^+$ ssRNA with P2 polymerase; and
c) subsequent purification of the newly formed dsRNA of the M segment through a 1 % agarose gel to achieve separation of said dsRNA from other components of the replication mixture.

According to some additional embodiments of the art, a set of single-stranded DNAs (exemplified by synthetic deoxyribooligonucleotides, M13 phage linear ssDNA) was shown to be replicable with P2 (Fig. 10A and not shown) under similar conditions as described above for single-stranded RNA. The reaction results in duplexes consisting of a template DNA and a newly produced RNA replica. Furthermore, some linear dsDNAs are shown to serve as the templates for the P2 catalyzed RNA-synthesis (Fig. 10B). And again, as in the case of RNA templates, single-stranded DNAs are much more efficient substrates than the double-stranded ones.

**Conditions for RNA synthesis on RNA and DNA substrates**

The method for producing RNA *in vitro* comprises

- providing RNA or DNA substrate;
- contacting said RNA or DNA substrate with the protein of the invention under conditions sufficient for RNA synthesis in a mixture comprising:

  (a) nucleic acid substrate, preferably 40 to 400 $\mu$g/ml;
  (b) protein of claims 1 to 5, preferably 0.005 to 0.5 mg/ml;
  (c) buffer, preferably pH 7.3-9.3;
  (d) $MgCl_2$ ions, preferably 0 to 10mM, more preferably 5 to 10mM;
  (e) nucleoside triphosphates, preferably 0.2 to 3 mM of each NTP;
  (f) PEG, preferably 0 to 9%;
  (g) ammonium acetate, preferably 0 to 200 mM;
  (h) $MnCl_2$, preferably 0 to 3mM;
  (i) BSA, preferably 0 to 1.0 mg/ml;

(j) DTT, preferably 0 to 5 mM
(k) a nonionic detergent, preferably 0 to 0.5 %

- incubating the reaction mixture in 20 to 42°C, and
- recovering the newly produced nucleic acid species from the reaction mixture.

**Method for amplifying RNA *in vitro***

This invention relates to a method for amplifying RNA *in vitro,* comprising the steps of:

(a) providing RNA substrate;
(b) contacting said RNA substrate with the protein of any one of the present invention under conditions sufficient for both RNA-replication and RNA-transcription; and
(c) recovering a mixture of the newly produced amplified RNA from the reaction mixture.

This invention relates furthermore to a method, comprising the steps of:

(a) providing ssRNA substrate;
(b) replicating said ssRNA substrate with the protein of the present invention to form dsRNA;
(c) transcribing said dsRNA with the protein of the present invention to obtain ssRNA; and
(d) repeating the amplification steps until a sufficient amount of RNA synthesis products have been obtained.

Interestingly, the P2-driven transcription of the synthetic M segment (Fig. 9, M) was carried out under conditions indistinguishable from those preferably used for the $m^+$ replication. This fact allows one to suggest that the dsRNA newly formed in the P2-catalyzed replication of a ssRNA substrate may serve as a template for the P2-catalyzed transcription. Such co-occurrence of replication and transcription in a single test tube implies that a reaction programmed with a single-stranded RNA substrate will result not only in dsRNA species (products of the input ssRNA replication) but also in ssRNA species (products of transcription of the newly formed dsRNA species). At least some of the newly synthesized ssRNAs will be of the same polarity as the input ssRNA substrate thus implying amplification of the input substrate in the P2-containing reaction mixture. In principle the newly produced ssRNA mentioned above might in turn undergo additional round or even several rounds of such amplification. Based on this scheme, the invention provides a method for the RNA amplification that consists of the steps of: a) providing RNA substrate; b) contacting this RNA with the polymerase protein of this invention; recovering the amplified RNA. Under the presently preferred conditions transcription of dsRNAs is somewhat less efficient than replication of ssRNAs as calculated using phosphoroimager analysis of the band intensities.

This invention relates also to a method for producing RNA *in vitro,* comprising the steps of

(a) providing ssRNA substrate by transcribing a DNA template with a DNA-dependent RNA polymerase; and
(b) replicating said ssRNA substrate with the protein of any one claims 1 to 5 to form dsRNA.

This method can be used in a method for amplifying RNA *in vitro,* comprising in addition the steps of:

(c) transcribing said dsRNA with the protein of any one of claims 1 to 5 to obtain ssRNA; and
(d) repeating the amplification steps until a sufficient amount of RNA synthesis products have been obtained.

In a specific embodiment of the present invention, ssRNA substrate for the P2-catalysed replication can be provided by transcribing DNA templates with a DNA-dependent RNA polymerase. In a preferred case, the DNA-dependent RNA polymerase is derived from a bacteriophage. It is most advantageous that the bacteriophage is selected from the group consisting of T7, T3, and SP6 bacteriophages. In some embodiments of the art, said transcribing a DNA template with a DNA-dependent RNA polymerase and P2-catalyzed replicating of the newly produced linear ssRNA can occur in the same reaction vessel. Special experiments were carried out in order to demonstrate possibility of the latter approach. In these experiments, linear dsDNA containing promoter for T7 RNA polymerase (namely, pLM659 cut with *Sma*I*)* was incubated with both T7 RNA polymerase and P2 RNA polymerase under condition essentially the same as described in Example 2 for P2-catalyzed RNA replication except temperature was 37°C. The reaction products comprised essentially the mixture of ssRNA and dsRNA migrating in the standard agarose gel-electrophoresis at the positions of correspondingly s+ and S segments of ϕ6 (not shown). Only ssRNA species was formed when P2 was omitted from the reaction mixture.

Based on the findings listed above, the present invention provides methods for producing RNA using polymerase of this invention contacted with different nucleic acid templates. Some of these methods are designed to be used for such special applications as increasing stability of nucleic acids, primer-independent sequencing, and primer extension.

**A method for stabilizing nucleic acids**

This invention relates to a method for stabilizing nucleic acids, comprising the steps of:

(a) providing single-stranded nucleic acid substrate;
(b) contacting said single-stranded nucleic acid substrate with the protein of the invention under conditions sufficient for RNA synthesis in order to convert at least part of the single-stranded nucleic acid substrate to the double-stranded nucleic acid form;
(c) recovering total nucleic acids from the reaction mixture;
the method may optionally comprise further steps of:
(d) contacting said total nucleic acids with a preparation containing nuclease or nucleases selectively degrading single-stranded nucleic acids but not double-stranded nucleic acids; and
(e) recovering the double-stranded nucleic acids showing increased stability to the degradation by nucleases.

The method of increasing stability of a single-stranded nucleic acid is based on the phenomenon that double-stranded nucleic acids are resistant to degradation by single-stranded specific nucleases under certain conditions (as illustrated for instance in Fig. 3C).

**Determining the nucleotide base sequence of a linear nucleic acid**

The present invention relates to a method for determining the nucleotide base sequence of a linear nucleic acid molecule, comprising the steps of:

(a) providing linear nucleic acid molecule;
(b) incubating said nucleic acid molecule under conditions sufficient for RNA synthesis in a mixture comprising:

- protein of the present invention;
- four nucleoside-triphosphates or functional analogs thereof; and
- at least one of four RNA synthesis terminating agents which terminate RNA synthesis at a specific nucleotide base,
  wherein each said agent terminates RNA synthesis at a different nucleotide base;
  and

(c) separating the terminated RNA products of the incubating reaction according to their size, whereby at least a part of the nucleotide base sequence of said nucleic acid molecule can be determined.

The method of primer-independent enzymatic sequencing of a nucleic acid relies on the fact that the polymerase of this invention (P2 protein in a preferred embodiment) can initiate RNA synthesis 1) without primers and 2) starting from the very 3' terminal nucleotide of a nucleic acid template (ssRNA in a preferred embodiment) (Fig. 7). Due to the latter feature, newly produced RNA chains will have uniform *5'* end in the case the template preparation is homogeneous. Advantageously, it has been demonstrated that the polymerase of this invention is able to incorporate 3'-deoxynucleotides into the growing RNA chain resulting in chain termination at specific positions (not shown). Several methods of nucleic acid sequencing based on the DNA or RNA polymerization reactions have been described in the previous art (e.g., U.S. Patent No. 5173411 and references therein; and Axelrod and Kramer, 1985). In all these methods, polymerization is caused to terminate at specific bases via incorporation of base-specific chain terminating agents, for example dideoxynucleotides (for DNA polymerases) or 3'-deoxynucleotides (for RNA polymerases). In the case of sequencing based on DNA polymerases, polymerization is initiated from a primer complementary to the template of interest. DNA-dependent RNA polymerases have been used to sequence DNA without primers (Axelrod et al., 1985). However in this case, DNA template has to contain a specific promoter for the initiation of RNA synthesis. Advantageously, the method of nucleic acid sequencing of present art requires neither primer nor promoter. The only limitation of this method is the presence of a free 3' end in the polynucleotide to be sequenced.

**Primer extension method**

This invention discloses also a method for primer extension using the polymerase of this invention. The method of primer extension is based on the observation made in a specific embodiment of this invention, where P2 was used to synthesize RNA in the presence of a nucleic acid template comprising essentially ssRNA template and a labeled deoxyribooligonucleotide primer complementary to an internal part of said template. It was shown that the P2 polymerase could extend primer by adding nucleotides to its 3' end. This type of RNA synthesis completely depended on the presence of the ssRNA template. The size of the major reaction product was consistent with the assumption that the RNA polymerization begins from the 3'-end of the primer and continues until the polymerase reaches the very 5'-end of the ssRNA template (not shown).

**Kit for the in *vitro* RNA synthesis and for sequencing**

The present invention also provides kits for the *in vitro* RNA synthesis. The kits include a preparation of the polymerase protein of this invention and additives necessary for an adequate level of the RNA synthesis. Possible nature of these additives is readily understood from the detailed description of RNA synthesis *in vitro* (Examples 2 and 3). The additives comprise typically buffers, salts, PEG and/or DTT. In a specific embodiment of the invention, the kit may contain nucleoside triphosphates and/or a nucleic acid preparation (or preparations) that has (have) been shown to stimulate detectable RNA synthesis. In another specific embodiment, nucleoside triphosphate mixture may also contain at least one nucleoside triphosphate modified to contain a detectable label.

The present invention discloses also a kit specifically used for sequencing. Said kit comprises at least one RNA syntheis terminating agent which terminate RNA synthesis at a specific nucleotide base.

**Incorporation of chemically modified nucleotides into the RNA product**

In addition to normal or radiolabeled nucleoside triphosphates, the inventors have shown that a polymerase of the invention incorporates chemically modified nucleotides into the RNA product. This incorporation makes possible an RNA synthesis assay that uses non-radioactive methodology, such as that based on fluorescence or chemiluminescence detection. RNA products containing fluorescent labels or other non-radioactive labels also can be used as RNA probes, for instance.

In a preferred embodiment, standard P2 replication mixtures containing a ssRNA substrate were supplemented with 0.02 to 0.1 mM of Alexa Fluor ® 488-5-UTP (Molecular Probes), coumarin-5-CTP (New England Nuclear), or biotin-11-CTP (New England Nuclear). Reactions were incubated for 1 hour at 30 °C. The reaction mixtures were then passed through AutoSeq G-50 spin columns (Pharmacia) to purify RNA products from the non-reacted nucleotide analogs and from the other low molecular weight contaminants. Incorporation of the nucleotide analogs into the newly produced RNA was then measured in the flow-through fractions using a spectrofluorometer (in the case of Alexa Fluor ® 488-5-UTP and coumarin-5-CTP) or a dot blot assay (for biotin-11-CTP). In each of these cases, a detectable part of the analog was found in the RNA product fraction (Fig. 11).

EXAMPLE 1

**Expression and purification of recombinant P2 polymerase of bacteriophage φ6**

*Construction of P2 producing strain*

To construct a plasmid for P2 protein expression, P2 gene (SEQ ID NO:1) was PCR-amplified from pLM687 (Mindich *et al.,* 1994) template with the recombinant *Pfu* DNA polymerase (Stratagen) and the oligonucleotides

5'-GGTAAGCGCCATATGCCGAGGAGA-3' (SEQ ID NO. 2) and 5'-TACGAATTCCGGCATGATTACCTAGGCATTACA-3' (SEQ ID NO. 3) serving as upstream and downstream primers respectively. The PCR fragment digested with *Nde*I and *Eco*RI (underlined sites in the primer sequences) was gel-purified and ligated with the large fragment of the *Nde*I-*Eco*RI cut vector pET32b(+) (Novagen). *E. coli* BL21(DE3) (Studier and Moffatt, 1986; purchased from Novagen) was transformed with the resultant plasmid pEM2 to give P2 producing strain BL21(DE3/pEM2). The sequence of the entire P2 insert was determined (SEQ ID NO:8). A single amino acid change, Ile457 to Met, was found when compared to the published protein sequence (GeneBank, AAA32355). Methionine codon at this position was also found in the plasmid pLM687 that had been used as a template for the gene amplification. This plasmid contains the cDNA copy of the entire large genomic segment of the φ6 phage and it has been previously employed in the reverse genetics experiments to produce viable virus particles (Mindich *et al.,* 1994). Thus, the observed change does not impair P2 activity in the virus.

*Expression and purification of recombinant P2 protein*

Purification of P2 protein was monitored by SDS-PAGE in 12.5% acrylamid gel (Olkkonen and Bamford, 1989) and by immunoblotting with rabbit polyclonal antibodies raised against recombinant polymerase complex (PC) particles (Frilander and Bamford, 1995). Strain BL21(DE3/pEM2) produced a detectable amount of the soluble P2 protein at 15 to 23°C as judged by SDS-PAGE and immunoblotting analysis (Fig. 1, lanes 1-3). Noteworthy, expression at 28 to 37°C led to much higher production of P2, with almost all of the synthesized protein in an insoluble form (not shown). To achieve expression of soluble P2, a starter culture of BL21 (DE3/pEM2) in the LB medium containing 150 mg/ml ampicillin was grown at 37°C with shaking until $OD_{540}$ reached 0.5. This was then diluted 50-fold into 3 L of the same medium. The diluted culture was further grown at 37°C up to $OD_{540}$ of 1.0. The culture was chilled on ice and induced with 1 mM of isopropyl β-D-thiogalactopyranoside (IPTG). IPTG induced cells were then transferred to 15°C where they were shaken for 18 h (see Fig. 1, lanes 1-2). Alternatively, expression was done at 20-23°C shaking the induced culture for 14h. All the following steps, unless otherwise indicated, were performed at 4°C. Bacteria were collected by centrifugation and resuspended in 30 ml of buffer Al (100 mM NaCl, 50 mM TrisHCl, pH 8.0, 1 mM EDTA). The suspension was passed 3 times at ~ 105 MPa through a precooled French pressure cell. Phenylmethylsulphonylfluoride was added to 1 mM after the first passage. The lysate was centrifuged at 120,000 g for 2 h 30 mm. Supernatant fraction (Fig. 1, lane 3) was loaded onto a dye affinity column (Cibacron Blue 3GA, Sigma). Proteins bound to the column were eluted with buffer AS (500 mM NaCl, 50 mM TrisHCl, pH 8.0, 1 mM EDTA). Pooled fractions containing P2 (Fig. 1, lane 4) were diluted fivefold with ice-cold distilled water and applied onto a heparin agarose column (Sigma). Proteins were eluted with a linear gradient of 0.1 to 1M NaCl buffered with 50 mM TrisHCl, pH 8.0 and 1 mM EDTA. Fractions containing P2 (Fig. 1, lane *5*) were pooled and diluted tenfold with 20 mM TrisHCl, pH 8.0, filtered and injected onto a Resource Q column (Pharmacia; room temperature). Elution of the bound proteins was performed with a gradient of 0 to 0.5 M NaCl buffered with 50mM TrisHCl, pH 8.0 and 0.1 mM EDTA. P2 eluted as a single peak at approximately 90-100 mM NaCl (Fig. 1, lane 6). The concentration of the purified P2 protein was determined by absorbance at 280 nm in 6 M guanidine hydrochloride (based on the value of 1.39 per 1 mg/ml calculated for the unfolded protein; Edelhoch, 1967). The estimated yield of the purified protein was about 1 mg per liter of the bacterial culture. Somewhat better yields were usually obtained when P2 was expressed at 20 to 23°C. Purified P2 was stored on ice for up to one month without detectable loss of activity or protein integrity.

EXAMPLE 2

**RNA-replication**

*Bacterial strains and plasmids*

*Escherichia coli* DH5α (Gibco-BRL) was the host for the plasmid propagation and molecular cloning. Plasmids pLM659 (Gottlieb *et al.,* 1992b), pLM656 (Olkkonen *et al*., 1990) and pLM687 (Mindich *et al.,* 1994) allowed production of the positive-sense ssRNA copies of the bacteriophage genomic segments $s^+$, $m^+$ and $1^+$, respectively. Plasmid pLM1809 (Qiao *et al.,* 1997) was used for synthesis of a long RNA containing fused $s^+$, $m^+$ and $1^+$ segments. Plasmid pGEMluc (Promega) was employed to produce *Photinus pyralis* luciferase mRNA. Plasmids pTU58 (Chalfie *et al.,* 1994) and pTZluc(NPT2) (Makeyev *et al.,* 1996) were the templates for production of mRNAs encoding green fluorescent protein and translational fusion of firefly luciferase and neomycin phosphotransferase II.

*Preparation of ssRNA substrates*

Synthetic single-stranded RNA substrates were prepared by *in vitro* transcription with SP6 (for pGEMluc) or T7 (for the rest of DNA templates) RNA polymerases. The unlabeled RNAs were produced in 50 μl transcription mixtures in principle as described in Makeyev *et al.,* 1996. The mixtures were incubated at 37°C for 2 h and then stopped by the addition of 1 unit of DNase RQ (Promega) per 1 μg of input DNA template. Incubation was continued for a further 15 mm at 37°C. RNA preparations were successively extracted with phenol/chloroform (1:1) and chloroform, precipitated with 3M LiCl and dissolved in sterile water. Labeled $m^+$ RNA was synthesized as recommended by Promega. The mixture (25 μl) contained 1 mCi/ml of [$\alpha^{32}$P]UTP (Amersham, 3000 Ci/mmol), 20 units of RNasin, 4 μg of pLM656 treated with *Xba*I (NEB) and mung bean nuclease (Promega), and 40 units of T7 RNA polymerase. The reaction was carried out for 1 h and then processed as described for unlabeled transcripts with the only exception that the labeled RNA was additionally purified by passing through a Sephadex G25 spin column (Pharmacia) after the LiCl precipitation step. A mixture of the natural ϕ6 transcripts (single-stranded segments $s^+$, $m^+$, and $1^+$) was prepared using nucleocapsid-directed transcription (Bamford *et al.,* 1995) followed by phenol extraction and three successive LiCl precipitations. The RNA concentration was measured by optical density at 260 nm. The quality of the RNAs was determined by electrophoresis either in 5 % polyacrylamide gel (PAAG) containing 7.5 M urea or in the standard 1% agarose gel (Pagratis and Revel, 1990).

*Assaying RNA synthesis in vitro*

The replication activity of P2 protein prepared as described in Example 1 was typically assayed in a 10 μl reaction mixture containing 50 mM Tris HC1, pH 8.9, 80 mM ammonium acetate ($NH_4OAc$), 6 % (w/v) PEG4000, 5 mM $MgCl_2$, 1 mM $MnCl_2$, 2 mM DTT, 0.1 mM EDTA, 1 mM each of ATP and GTP, 0.2 mM each of CTP and UTP (all four nucleotide triphosphates from Pharmacia), 0.2 mg/ml BSA (nuclease free, NEB), and 0.8 u/μl RNasin. The final concentration of the added RNA substrates ranged from 40 μg/ml to 300 μg/ml. Unless indicated otherwise, the mixture was supplemented with 0.25-0.5mCi/ml of [$\alpha^{32}$P]UTP (Amersham, 3000 Ci/mmol). Reactions were initiated by addition of 0.2-2 μl of the P2 protein preparation. In the control reactions ("buffer only"), P2 was replaced with an equal volume of the P2 buffer (50 mM Tris HCl, pH 8.0, 90 mM NaCl, 0.1 mM EDTA, 0.2 mg/ml BSA). The mixtures were incubated at 28°C for 1 h and processed for further analysis. Two types of agarose gel-electrophoresis, both originally described by Pagratis and Revel (1990), were employed in this study for the RNA analysis. The first, or standard, type of electrophoresis used to achieve separation of the positive-sense ssRNA and the corresponding dsRNA segments, was carried out in 1% agarose gels containing 0.25 μg/ml of EtBr and buffered with 1xTBE (50 mM Tris-borate, pH 8.3, 1 mM EDTA). For analysis of the P2 polymerization products, the reaction was stopped by the addition of an equal volume of U2 buffer (8M Urea, 10 mM EDTA, 0.2% SDS, 6% (v/v) glycerol, 0.05% bromophenol blue and 0.05% xylene cyanol FF). After the RNA separation(5 V/cm), gels were irradiated with UV light and photographed. To determine the position of the radioactively labeled bands, gels were dried and exposed with Fuji Super RX film. Fig. 2 and Fig. *5* show typical results of the P2-catalyzed RNA replication as analyzed by the standard electrophoresis.

The second technique was the strand-separating gel analysis. In this case, electrophoresis was done in 1 % agarose buffered with 1xTBE and containing no EtBr. Samples for the analysis were prepared by stopping P2 reaction mixtures with 4 volumes of 100 mM EDTA, followed by phenol/chloroform (1:1) and chloroform extractions. The aqueous phase was made 2.5 M in $NH_4OAc$ and precipitated with 2.5 volumes of ethanol. The pellets were dissolved in U2 buffer diluted twofold with sterile water. When appropriate, the samples were boiled for 3 min and then placed on ice for another 3 min. After the RNA separation (5 V/cm), gels were stained with EtBr and processed as indicated for the standard gels. The strand-separating analysis was used to reveal the nature of the newly synthesized RNA product. Unless heat-treated, the radioactive product of the P2-catalyzed reaction programmed with $m^+$ template migrated in the strand-separating gel at the position of double-stranded M segment (Fig. 3A and B), as was found in the previous experiment. However, the product mobility changed after the heat-denaturation step to that of the minus-strand ($m^-$) of M segment. Thus we concluded that the P2 protein catalyzed the synthesis of the minus-strand complementary to the input plus-strand template, i.e. the replication reaction.

*RNase protection assay*

In the next experiment we checked whether the duplex of the RNA substrate and newly synthesized strand possessed properties of a dsRNA molecule. The corresponding experiment was based on the fact that the RNase I of *E. coli* readily hydrolyzes single-stranded and partially double-stranded RNA but not the perfect RNA duplexes (Brewer *et al*., 1992). The RNase protection assay was performed in 10 μl reaction mixtures containing 10 mM Tris HCl (pH 7.5), 200 mM $NH_4OAc$, 5 mM EDTA, 1 unit of RNase I (RNase ONE; Promega) and the RNA sample purified with phenol/chloroform extraction and ethanol precipitation from the P2 polymerase assay mixture. The reaction was carried out for 1 h at 28°C and stopped by the addition of 0.1 % SDS and 10 μg of *E. coli* tRNA (Sigma). The products of the reaction were analyzed by standard electrophoresis in agarose gel. As evident from the results shown in Fig. 3C, the replication product (duplex of $m^+$ and $m^-$) was almost fully resistant to the RNase digestion, whereas the RNA substrate ($m^+$) was completely degraded under the same conditions. Thus, the replication product represented the perfect double-stranded RNA composed by complementary $m^+$ and $m^-$ strands.

*Analytical gel filtration*

Direct association of the RNA-synthesizing activity with P2 protein was shown using non-denaturing gel-filtration. Chromatography was performed at room temperature on a Superdex 75 HR 10/30 column (Pharmacia) using buffer containing 50 mM Tris HCl, 100 mM NaCl and 0.1 mM EDTA and a flow rate of 0.5 ml/min. The proteins and the Blue Dextran used for calibration were from Sigma except for the purified mouse IgG (Zymed) and soybean trypsin inhibitor (Boebringer). Typically, 200 μg of purified P2 was injected onto the column and 0.5 ml fractions were collected. One microliter aliquots from each of the fractions were assayed for replicase activity with the $m^+$ RNA substrate as described above. As a result, P2 was found to migrate as a single peak with an apparent molecular mass of 45 kDa (Fig. 4), whereas the actual molecular mass of P2 is 75 kDa. This difference could not be explained by protein degradation (see SDS-PAGE in Fig. 1 and 4A). Possible interaction of the protein to the gel-filtration matrix (Sephadex) also seemed an unlikely explanation, because a similarly low apparent molecular weight was obtained with a different column (Ultrahydrogel 500, Waters, not shown). Therefore it is reasonable to propose that the protein is a very compact spherical monomer in solution. This conclusion was further confirmed by preliminary light-scattering data (R. Tuma, unpublished results). The polymerase activity was only found in the protein peak thus indicating that P2 possesses the RNA polymerase activity by itself and the activity is associated with P2 monomer.

*P2 initiation site and elongation rate*

Some additional approaches have been used to characterize the initiation and elongation of the P2-driven replication *in vitro.*

(a) The primer extension assay (Fig. 7) showed that at least in the case of full-length $s^+$ segment, purified P2 initiates replication from the very 3' terminal nucleotide of the template as in actual $\phi$6 replication *in vivo.* The assay was done in 10 $\mu$l reaction mixtures containing 50 mM Tris HCl (pH 8.3), 50 mM KC1, 10 mM $MgCl_2$, 10 mM DTT, 0.5 mM spermidine, 0.6 mM each of the four deoxynucleotide triphosphates, and 5 units of AMV reverse transcriptase (Promega). As a primer, the reaction contained 0.5 pmol of oligonucleotide (5'-GGATAAACAAGTCCTTGTATAAC-3') (SEQ ID NO. 4) terminally labeled with polynucleotide kinase (Promega) and [$\gamma^{32}$P]ATP (Amersham, 3000 Ci/mmol). The primer was designed to be complementary to the minus-strand of the small $\phi$6 genome segment (s). Denatured RNA for the assay was prepared as follows: the standard 10 $\mu$l replication mixtures containing P2 polymerase or the P2 control buffer and lacking labeled nucleotides were extracted with phenol/chloroform (1:1) and chloroform, brought to 2.5 M $NH_4OAc$ and precipitated with ethanol. The RNA pellets were dissolved in sterile water, heated at 100°C for 3 min, chilled on ice for another 3 min, and transferred to room temperature. The RNA samples were mixed with the rest of the assay components and the mixtures were incubated at 42°C for 10 min. Reaction was stopped by adding 7.5 $\mu$l of 95 % formamide, 20 mM EDTA, 0.05 % bromophenol blue and 0.05% xylene cyanol FF. The stopped mixtures were then incubated at 80°C for 5 min and analyzed in a 6 % PAAG containing 7.5 M urea.

(b) A kinetic experiment was designed to determine the elongation rate of the replicating P2 polymerase. Replication of the natural $\phi$6 transcripts was initiated by adding P2 protein to the mixture, and aliquots were sampled at different time points for the subsequent electrophoretic analysis. As evident from the autoradiogram shown in Fig. 6A, the full-length S product appeared first after a short lag period, followed successively by M and L. The band intensities then increased at least to the 1 h (3600 s) point. The accumulation of individual dsRNAs in time were also plotted as the time course curves (Fig. 6 B-D). Extrapolating the linear phases of the curves to the time axis, we obtain characteristic times necessary for the complete synthesis of each double-stranded product. Assuming an even initiation on all three ssRNA species, the average elongation rate ($V_{av}$) can be calculated as:

$$V_{av} = [(L\text{-}M)/(\tau_L\text{-}\tau_M) + (M\text{-}S)/(\tau_M\text{-}\tau_S) + (L\text{-}S)/(\tau_L\text{-}\tau_S)]/3,$$

where L, M and S are the lengths of the corresponding segments (S=2948 bp, M=4063 bp and L=6374 bp; McGraw *et al.,* 1986; Gottlieb *et al.,* 1988; Mindich *et al.*, 1988); $\tau_L$,$\tau_M$ and $\tau_S$ are the observed characteristic times (Fig. 6B-D). Consequently, the elongation rate of P2 under tested conditions was approximately 120 bp/s.

## EXAMPLE 3

### RNA-synthesis in the reaction mixtures programmed with dsRNA, ssDNA and dsDNA substrates

*Nucleic acid preparations*

Double-stranded RNA substrates were prepared by phenol-chloroform extracted from the purified dsRNA viruses (bacteriophage $\phi$6, BTV, CPV, *Saccharomyces cerevisiae* virus L-A). The RNA was precipitated with ethanol and dissolved in sterile water. Great care was taken in the case of BTV and CPV to ensure the RNA preparation did not contain infectious - virus particles. Short linear single-stranded DNA substrates (deoxyribooligonucleotides) were prepared by chemical synthesis. Specifically, oligonucleotides:

5'-CGTTCAGTTCTCAGTTCT-3' (SEQ ID NO:5);
5'-GGTAAGCGCCATATGCCGAGGAGA-3'(SEQ ID NO:6); and
5'-CTGAATTCTAATACGACTCACTATAGATCCGACCGTAG-3' (SEQ ID NO:7) were used in this example. Long ssDNA of a recombinant bacteriophage M13 (M13mp10, Amersham) linearized with restriction endonuclease *Hin*fI was also used as a substrate for the RNA synthesis. Linear dsDNA substrates were prepared by cutting circular DNA of plasmid pUC 18 with the different restriction endonucleases: *Bam*HI, *Hinc*II, *Hind*III, *Kpn*I, *Pst*I, *Sac*I, *Sma*I and *Xba*I. Incubation with a restriction enzyme was always followed by phenol-chloroform extraction and ethanol precipitation. Concentrations of both dsRNA and DNA were measured by optical density at 260 nm. The quality of the nucleic acid preparations was determined by electrophoresis in 1 % agarose gel (Pagratis and Revel, 1990) or polyacrylamide gels containing 7.5 M Urea.

*RNA-synthesis assay*

The assay was performed essentially as described in Example 2 with the only exception that 40-300 μg/ml of a dsRNA, or 40-100 μg/ml of ssDNA, or 100 μg/ml of a dsDNA substrate was added to the reaction mixture instead of ssRNA. The mixture containing a nucleic acid substrate, P2 polymerase and all required additives was typically incubated at 28°C for 1h and the reaction products were analyzed by electrophoresis in either normal (Fig. 8, Fig. 9A, Fig. 10) or strand-separating (Fig. 9B) gels done as described in Example 2. After electrophoresis, gels were dried and exposed with Fuji Super RX film. P2 polymerase synthesizes RNA in the presence of various dsRNA templates (Fig. 8). Notably, plus-sense RNA strands are the major products of RNA synthesis on ϕ6 dsRNA (Fig. 9). This reminds situation with ϕ6 transcription *in vivo*. Incubation of the linear ssDNA of bacteriophage M13 in the presence of P2 polymerase (Fig. 10A, lane N) gives rise to a reaction product migrating as a double-stranded nucleic acid species of the corresponding size (approximately 7 kb). No labeled product appears in the control reactions without P2 (Fig. 10A, lane N) or containing only UTP instead of the mixture of the four nucleoside triphosphates (not shown). These data strongly suggest that the product occurs as a duplex of the template DNA and the newly synthesized RNA strand. Analogously, formation of the labeled DNA-RNA duplexes has been also demonstrated when the reaction mixture was programmed with short synthetic deoxyribooligonucleotides (not shown). Thus, the P2-catalyzed RNA synthesis on ssDNA templates most probably reminds the reaction with ssRNA templates. Incubation of some linear dsDNA templates with P2 results in appearance of labeled nucleic acid forms migrated as the input dsDNAs (Fig. 10B). Notably, efficiency of the RNA synthesis depends on the nature of dsDNA ends. pUC18 DNA cut with *Bam*HI*, Hin*III, *Pst*I, *Sac*I*, Sma*I or *Xba*I stimulated detectable incorporation of the labeled nucleotide, whereas the same DNA cut with *Hinc*II or *Kpn*I did not. No labeled product was detected in the control reactions without P2 or containing only UTP instead of the mixture of the four nucleoside triphosphates. It can be proposed that reaction on the dsDNA templates reminds dsRNA transcription.

*References Cited*

Axelrod,V.D. and Kramer,F.R. (1985) Transcription from bacteriophage T7 and SP6 RNA polymerase promoters in the presence of 3'-deoxyribonucleoside 5'-triphosphate chain terminators. Biochemistry, 24, 5716-5723.

Bamford,D.H., Ojala,P.M, Frilander,M., Walin,L. and Bamford,J.K.H. (1995) Isolation, purification, and function of assembly intermediates and subviral particles of bacteriophages PRD1 and ϕ6. In Adolph,K. W: (ed.) Methods in Molecular Genetics, vol. 6, Academic Press, pp. 455-474.

Blumenthal,T. (1980) Qβ replicase template specificity: different templates require different GTP concentrations for initiation. Proc. Natl. Acad. Sci. USA, 77, 2601-2605.

Bruenn,J.A. (1991) Relationships among the positive strand and double-strand RNA viruses as viewed through their RNA-dependent RNA polymerases. Nucleic Acids Res., 19. 217-226.

Bruenn,J.A. (1993) A closely related group of RNA-dependent RNA polymerases from double-stranded RNA viruses. Nucleic Acids Res., 21, 5667-5669.

Brewer,G., Murray,E. and Staeben,M. (1992) RNase ONE: Advantages for nuclease protection assays. Promega Notes Magazine, 38, 1-7.

Butcher,S.J., Dokland,T., Ojala,P.M., Bamford,D.H. and Fuller,S.D. (1997) Intermediates in the assembly pathway of the double-stranded RNA virus ϕ6. EMBO J., 16, 4477-4487.

Casini,G., Qiao,X. and Mindich,L. (1994) Reconstitution of active replicase in procapsids of the segmented dsRNA bacteriophage ϕ6. Virology, 204, 251-253.

Cohen,J. (1975) Ribonucleic acid polymerase activity in purified infectious pancreatic necrosis virus of trout. Biochem. Biophys. Res. Commun., 62, 689-695.

Chalfie,M., Tu,Y., Euskirchen,G., Ward,W.W. and PrasherD.C. (1994) green fluorescent protein as a marker for gene expression. Science, 263, 802-805.

Chen,D., Zeng,C.Q., Wentz,M.J., Gorziglia,M., Estes,M.K. and Ramig,R.F. (1994) Template-dependent, in vitro replication of rotavirus RNA. J. Virol., 68, 7030-7039.

de Haas, F., Paatero, A.O., Mindich, L., Bamford, D.H. and Fuller, S.D. (1999) A symmetry mismatch at the site of RNA packaging in the polymerase complex of dsRNA bacteriophage ϕ6. J. Mol. Biol., 294, 357-372.

Dreher,T.W. and Hall,T.C. (1988) Mutational analysis of the sequence and structural requirements in brome mosaic virus RNA for minus strand promoter activity. J. Mol. Biol., 201, 31-40.
Edelhoch,H. (1967) Spectroscopic determination of tryptophan and tyrosine in proteins. Biochemistry, 6, 1948-1954.
Fields,B.N. and Knipe,D.M. (1990) Fields Virology, Second Edition. Raven Press, New York.
Frilander,M., Gottlieb,P., Strassman,J., Bamford,D.H. and Mindich,L. (1992) Dependence of minus-strand synthesis on complete genomic packaging in the double-stranded RNA bacteriophage ϕ6. J. Virol., 66, 5013-5017.
Frilander,M. and Bamford,D.H. (1995) In vitro packaging of the single-stranded RNA genomic precursors of the segmented double-stranded RNA bacteriophage ϕ6: the three segments modulate each other's packaging efficiency. J. Mol. Biol., 246, 418-428.
Fujimura,T., Esteban,R. and Wickner,R.B. (1986) In vitro L-A double-stranded RNA synthesis in virus-like particles from Saccharomyces cerevisiae. Proc. Natl. Acad. Sci. USA, 83, 4433-4437.
Fujimura,T. and Wickner,R.B. (1988) Replicase of L-A virus-like particles of Saccharomyces cerevisiae. In vitro conversion of exogenous L-A and M1 single-stranded RNAs to double-stranded form. J. Biol. Chem., 263, 454-460.
Fujimura,T. and Wickner,R.B. (1989) Reconstitution of template-dependent in vitro transcriptase activity of a yeast double-stranded RNA virus. J. Biol. Chem., 264, 10872-10877.
Gottlieb,P., Metzger,S., Romantschuk,M., Carton,J., Strassman,J., Bamford,D.H., Kalkkinen,N. and Mindich,L. (1988) Nucleotide sequence of the middle dsRNA segment of bacteriophage ϕ6: placement of the genes of membrane-associated proteins. Virology, 163, 183-190.
Gottlieb,P., Strassman,J., Qiao,X.Y., Frucht,A. and Mindich,L. (1990) In vitro replication, packaging, and transcription of the segmented double-stranded RNA genome of bacteriophage ϕ6: studies with procapsids assembled from plasmid-encoded proteins. J. Bacteriol., 172, 5774-5782.
Gottlieb,P., Strassman,J. and Mindich,L. (1992a) Protein P4 of the bacteriophage ϕ6 procapsid has a nucleoside triphosphate-binding site with associated nucleoside triphosphate phosphohydrolase activity. J. Virol., 66, 6220-6222.
Gottlieb,P., Strassman,J., Qiao,X., Frilander,M., Frucht,A. and Mindich,L. (1992b) In vitro packaging and replication of individual genomic segments of bacteriophage ϕ6 RNA. J. Virol., 66, 2611-2616.
Herring,A.J. and Bevan,E.A. (1977) Yeast virus-like particles possess a capsid-associated single-stranded RNA polymerase. Nature (London), 268, 464-466.
Joklik,W.K. (1974) Reproduction of the reoviridae. In Fraenkel-Conrat,H. and Wagner,R.R. (eds) Comprehensive Virology, vol. 2, Plenim Press, New York, pp. 231-334.
Juuti,J.T. and Bamford,D.H. (1995) RNA binding, packaging and polymerase activities of the different incomplete polymerase complex particles of dsRNA bacteriophage ϕ6. J. Mol. Biol., 249, 545-554.
Juuti,J.T. and Bamford,D.H. (1997) Protein P7 of phage ϕ6 RNA polymerase complex, acquiring of RNA packaging activity by in vitro assembly of the purified protein onto deficient particles. J. Mol. Biol., 266, 89 1-900.
Juuti,J.T., Bamford,D.H., Tuma,R. and Thomas,G.J.Jr (1998) Structure and NTPase activity of the RNA-translocating protein (P4) of bacteriophage ϕ6. J. Mol. Biol., 279, 347-359.
Koonin,E.V., Gorbalenya,A.E. and Chumakov,K.M. (1989) Tentative identification of RNA-dependent RNA polymerases of dsRNA viruses and their relationship to positive strand RNA viral polymerases. FEBS Lett., 252, 42-46.
Macreadie,I.G. and Azad,A.A. (1993) Expression and RNA dependent RNA polymerase activity of birnavirus VP1 protein in bacteria and yeast. Biochem. Mol. Biol. Int., 30, 1169-1178.
Makeyev,E.V., Kolb,V.A. and Spirin,A.S. (1996) Enzymatic activity of the ribosome-bound nascent polypeptide. FEBS Lett., 378, 166-170.
McGraw,T., Mindich,L. and Frangione,B. (1986) Nucleotide sequence of the small double-stranded RNA segment of bacteriophage ϕ6: novel mechanism of natural translational control. J. Virol., 58, 142-151.
Mindich,L., Nemhauser,I., Gottlieb,P., Romantschuk,M., Carton,J., Frucht,S., Strassman,J., Bamford,D.H. and Kalkkinen,N. (1988) Nucleotide sequence of the large double-stranded RNA segment of bacteriophage ϕ6: genes specifying the viral replicase and transcriptase. J. Virol., 62, 1180-1185.
Mindich,L., Qiao,X., Onodera,S., Gottlieb,P. and Frilander,M. (1994) RNA structural requirements for stability and minus-strand synthesis of in the dsRNA bacteriophage ϕ6. Virology, 202, 258-263.
Mindich,L., Qiao,X., Qiao,J., Onodera,S., Romantschuk,M. and Hoogstraten,D. (1999) Isolation of additional bacteriophages with genomes of segmented double-stranded RNA. J. Bacteriol., 181, 4505-4508.
Olkkonen,V.M. and Bamford,D.H. (1989) Quantitation of the adsorption and penetration stages of bacteriophage ϕ6 infection. Virology, 171, 229-238.
Olkkonen,V.M., Gottlieb,P., Strassman,J., Qiao,X.Y., Bamford,D.H. and Mindich,L. (1990) In vitro assembly of infectious nucleocapsids of bacteriophage ϕ6: Formation of a recombinant double-stranded RNA virus. Proc. Natl. Acad. Sci. USA, 87, 9173-9177.
Paatero,A.O., Syvaoja,J.E. and Bamford,D.H. (1995) Double-stranded RNA bacteriophage ϕ6 protein P4 is an unspecific nucleoside triphosphatase activated by calcium ions. J. Virol., 69, 6729-6734.
Paatero,A.O., Mindich,L. and Bamford,D.H. (1998) Mutational analysis of the role of nucleoside triphosphatase P4 in the assembly of the RNA polymerase complex of bacteriophage ϕ6. J. Virol., 72, 10058-10065.
Pagratis,N. and Revel,H.R. (1990) Detection of bacteriophage ϕ6 minus-strand RNA and novel mRNA isoconformers synthesized in vivo and in vitro, by strand-separating agarose gels. Virology, 177, 273-280.
Partridge,J.E., Van Etten,J.L., Burbank,D.E. and Vidaver,A.K. (1979) RNA polymerase associated with bacteriophage ϕ6 nucleocapsid. J. Gen. Virol., 43, 299-307.
Patton,J.T. (1996) Rotavirus VP1 alone specifically binds to the 3' end of viral mRNA, but the interaction is not sufficient to initiate minus-strand synthesis. J. Virol., 70, 7940-7947.
Patton,J.T., Jones,M.T., Kalbach,A.N., He,Y.W. and Xiaobo,J. (1997) Rotavirus RNA polymerase requires the core shell protein to synthesize the double-stranded RNA genome. J. Virol., 71, 9618-9626.
Qiao,X., Qiao,J. and Mindich,L. (1997) Stochiometric packaging of the three genomic segments of double-stranded RNA bacteriophage ϕ6. Proc. Natl. Acad. Sci. USA, 94, 4074-4079.
Sambrook et al. (1989) Molecular Cloning, A Laboratory Manual 2nd Edition, Cold Spring Harbor Laboratory Press, Cold Spring Harbor, NY.
Semancik,J.S., Vidaver,A.K. and Van Etten,J.L. (1973) Characterization of segmented double-helical RNA from bacteriophage ϕ6. J. Mol. Biol., 78, 617-625.
Sharp,P.A. (1999) RNAi and double-strand RNA. Genes Dev., 13, 139-141.
Studier,F.W. and Moffatt,B.A. (1986) Use of bacteriophage T7 RNA polymerase to direct selective high-level expression of cloned genes. J. Mol. Biol., 189, 113-130.
Urakawa,T., Ritter,D.G. and Roy,P. (1989) Expression of largest RNA segment and synthesis of VP1 protein of bluetongue virus in insect cells by recombinant baculovirus: association of VP1 protein with RNA polymerase activity. Nucleic Acids Res., 17, 7395-7401.
Valenzuela,S., Pizarro,J., Sandino,A.M., Vasquez,M., Femandez,J., Hernandez,O., Patton,J. and Spencer,E. (1991) Photoaffinity labeling of rotavirus VP1 with 8-azido-ATP: identification of the viral RNA polymerase. J. Virol., 65, 3964-3967.
Van Etten,J.L., Vidaver,A.K., Koski,RK., Semancik,J.S. (1973), RNA polymerase activity associated with bacteriophage ϕ6. J. Virol., 12, 464-47 1.
Van Etten,J.L., Vidaver,A.K., Koski,R.K. and Bumett,J.P. (1974) Base composition and hybridization studies of the three double-stranded RNA segments of bacteriophage ϕ6. J. Virol., 13, 1254-1262.
Van Dijk,A.A., Frilander,M. and Bamford,D.H. (1995) Differentiation between minus- and plus-strand synthesis: polymerase activity of dsRNA bacteriophage phi 6 in an in vitro packaging and replication system. Virology, 211, 320-323.
Vidaver,A.K., Koski,R.K. and Van Etten,J.L. (1973) Bacteriophage ϕ6: a lipid-containing virus of Pseudomonas phaseolicola. J. Virol., 11, 799-805.
Zeng,C.Q., Wentz,M.J., Cohen,J., Estes,M.K. and Ramig,R.F. (1996) Characterization and replicase activity of double-layered and single-layered rotavirus-like particles expressed from baculovirus recombinants. J. Virol., 70, 2736-2742.
US 5,173,411
US 5,614,403
US 5,631,129
US 5,795,715
US 5,854,037

## Claims

1. An isolated RNA-dependent RNA polymerase protein, wherein the protein has, in isolated form not assisted with any other proteins, an unspecific, primer-independent capability of RNA synthesis *in vitro* when contacted with nucleic acid substrates under sufficient conditions, said protein originating from a *Cystoviridae-* family virus and said polymerase being able to catalyze RNA synthesis in the presence of ssRNA, dsRNA, ssDNA and dsDNA substrates.

2. The protein according to claim 1, wherein the protein originates from ϕ6-related bacteriophages.

3. The protein according to claim 1 or 2, wherein the protein originates from ϕ6, ϕ7, ϕ8, ϕ9, ϕ10, ϕ11, ϕ12, ϕ13 or ϕ14.

4. The protein according to any one of claims 1 to 3, wherein the protein is P2 or an altered or a genetically modified form of P2, having in isolated form not assisted with any other proteins an unspecific primer-independent capability of RNA synthesis *in vitro*.

5. The protein according to any one of claims 1 to 4 selected from the group comprising:

   a) a protein encoded by a nucleic acid sequence having a nucleic acid sequence of SEQ ID NO:1;
   b) a protein encoded by a nucleic acid sequence encoding a polypeptide having an amino acid sequence of SEQ ID NO:8; and
   c) an amino acid sequence which shows at least 80% identity to the amino acid sequences SEQ ID NO:8.

6. The protein according to any one of claims 1 to 5, wherein the protein comprises the amino acid sequence SEQ ID NO:8.

7. A method for producing the protein of any one of claims 1 to 6 comprising the steps of:

   (a) culturing host cells containing a nucleic acid sequence encoding the protein of any one of claims 1 to 6 in a culture medium to express said protein in soluble form;
   (b) recovering the protein from the host or from the culture medium;
   (c) purifying and isolating the protein from other proteins; and optionally

(d) assaying the RNA-synthesizing activity of said protein.

**8.** A method for isolating and purifying the protein according to claim 7, wherein the method comprises the further steps of:

a) disrupting the host cells in a buffer to obtain a cell lysate;
b) clarifying said lysate by centrifugation;
c) purifying the protein using at least one step, more preferably two steps of affinity chromatography;
d) further purifying the protein using at least one step of ion exchange chromatography to obtain a fraction that is essentially free of nuclease and protease activities.

**9.** A method for producing RNA *in vitro,* comprising the steps of:

- providing ssRNA or dsRNA substrate;
- contacting said ssRNA or dsRNA substrate with the protein of any one of claims 1 to 6 in isolated form under conditions sufficient for RNA synthesis; and
- recovering the newly produced RNA species from the reaction mixture.

**10.** The method of claim 9, wherein said newly produced RNA species is dsRNA.

**11.** The method of claim 9 or 10, comprising the following steps for amplifying RNA *in vitro* :

(a) providing said RNA substrate;
(b) contacting said RNA substrate with the protein of any one of claims 1 to 6 in isolated form under conditions sufficient for both RNA-replication and RNA-transcription; and
(c) recovering a mixture of the newly produced amplified RNA from the reaction mixture.

**12.** The method of claim 11, comprising the steps of:

(a) providing ssRNA substrate;
(b) replicating said ssRNA substrate with the protein of any one of claims 1 to 6 in isolated form to form dsRNA;
(c) transcribing said dsRNA with the protein of any one of claims 1 to 6 to obtain ssRNA; and
(d) repeating the amplification steps until a sufficient amount of RNA synthesis products have been obtained.

**13.** The method of claim 9 or 10, comprising the steps of:

(a) providing ssRNA substrate by transcribing a DNA template with a DNA-dependent RNA polymerase; and
(b) replicating said ssRNA substrate with the protein of any one of claims 1 to 6 in isolated form to form dsRNA.

**14.** The method according to claim 13, further comprising the following steps for amplifying RNA *in vitro*:

(c) transcribing said dsRNA with the protein of any one of claims 1 to 6 in isolated form to obtain ssRNA; and
(d) repeating the amplification steps until a sufficient amount of RNA synthesis products have been obtained.

**15.** The method of claim 13 or 14, wherein said DNA dependent RNA polymerase is derived from a bacteriophage, preferably selected from the group comprising T7, T3, and SP6 bacteriophages.

**16.** The method of any one of claims 13 to 15, wherein steps (a) and (b) are carried out at the same time or sequentially in the same reaction vessel.

**17.** A method for stabilizing nucleic acids, comprising the steps of:

(a) providing single-stranded nucleic acid substrate;
(b) contacting said single-stranded nucleic acid substrate with the protein of any one of claims 1 to 6 in isolated form under conditions sufficient for RNA synthesis in order to convert at least part of the single-stranded nucleic acid substrate to the double-stranded nucleic acid form;
(c) recovering total nucleic acids from the reaction mixture; and
(d) contacting said total nucleic acids with a preparation containing nuclease or nucleases selectively degrading single-stranded nucleic acids but not double-stranded nucleic acids; and
(e) recovering the double-stranded nucleic acids showing increased stability to the degradation by nucleases.

**18.** A method for producing RNA *in vitro,* comprising the steps of:

(a) providing an ssDNA or dsDNA substrate;
(b) contacting said ssDNA or dsDNA substrate with the protein of any one of claims 1 to 6 in isolated form under conditions sufficient for RNA synthesis; and
(c) recovering the newly produced nucleic acid species from the reaction mixture.

**19.** The method of claim 18, wherein the newly produced nucleic acid species comprises duplexes consisting of template DNA and RNA replica.

**20.** The method of any one of claims 9 to 19, wherein the single-stranded or double-stranded nucleic acid substrate is linear.

**21.** The method of any one of claims 9 to 20, wherein the mixture for RNA synthesis contains at least one nucleoside triphosphate labeled with a radioactive isotope or is chemically modified.

**22.** A method for producing RNA *in vitro,* comprising the steps of:

- providing RNA or DNA substrate;
- contacting said RNA or DNA substrate with the protein of any one of claims 1 to 6 in isolated form under conditions sufficient for RNA synthesis in a mixture comprising: nucleic acid substrate, protein of any one of claims 1 to 6, nucleoside triphosphates, and optionally buffer, ammonium acetate, DTT, PEG, $Mg^{2+}$-ions, $Mn^{2+}$-ions and/or BSA; and
- incubating the reaction mixture at temperature sufficient for RNA synthesis;
- recovering the newly produced nucleic acid species from the reaction mixture.

**23.** The method of any one of claims 9 to 22, wherein RNA synthesis is initiated from the 3' end of a primer complementary to the nucleic acid substrate.

**24.** The method of claim 23, wherein said primer is single-stranded RNA or DNA.

**25.** A kit for producing RNA *in vitro* comprising:

(a) a polymerase protein of any one of claims 1 to 6 in isolated form ;and optionally
(b) additives necessary for a detectable level of RNA synthesis.

**26.** The kit of claim 25 comprising nucleoside triphosphates in concentrations sufficient for RNA synthesis.

**27.** The kit of claim 25 or 26, wherein at least one nucleoside triphosphate is labeled with a radioactive isotope or is chemically modified.

**28.** The kit of any one of claims 25 to 27, wherein the kit additionally contains a standard nucleic acid preparation (or preparations) with **characterized** capacity to serve as a template (templates) for RNA synthesis.

**29.** The kit of any one of claims 25 to 28 specifically used for sequencing nucleic acid molecule and optionally comprising at least one RNA synthesis terminating agent which terminate RNA synthesis at a specific nucleotide base.

**30.** The kit of claim 29, wherein said RNA synthesis terminating agent is a 3'-deoxynucleoside triphosphate or a functional derivative thereof.

**31.** A method for determining the nucleotide base sequence of a linear nucleic acid molecule, comprising the steps of:

(a) providing linear nucleic acid molecule;
(b) incubating said nucleic acid molecule under conditions sufficient for RNA synthesis in a mixture comprising:

- protein of any one of claims 1 to 6 in isolated form;
- four nucleoside-triphosphates or functional analogs thereof; and
- at least one of four RNA synthesis terminating agents which terminate RNA synthesis at a specific nucleotide base,

wherein each said agent terminates RNA synthesis at a different nucleotide base; and

(c) separating the terminated RNA products of the incubating reaction according to their size, whereby at least a part of the nucleotide base sequence of said nucleic acid molecule can be determined.

**32.** The method of claim 31, wherein said nucleic acid molecule is single-stranded RNA or DNA.

**33.** The method of claim 31, wherein said nucleic acid molecule is double-stranded RNA or DNA.

**34.** The method of any one of claims 31 to 33, comprising use of at least one of said nucleoside-triphosphates or functional analogs thereof modified to contain detectable label.

**35.** The method of any one of claims 31 to 34, comprising use of at least one of said RNA synthesis terminating agents modified to contain detectable label.

**36.** The method of any of claims 31 to 35, wherein said RNA synthesis terminating agents are 3'-deoxynucleoside triphosphates or functional derivatives thereof.

**Patentansprüche**

**1.** Isoliertes RNA-abhängiges RNA-Polymerase-Protein, wobei das Protein, welches in isolierter Form nicht von anderen Proteinen unterstützt wird, eine unspezifische, Primer-unabhängige Fähigkeit zur RNA-Synthese *in vitro* hat, wenn es unter ausreichenden Bedingungen in Kontakt mit Nucleinsäuresubstraten gebracht wird, wobei das Protein aus einem Virus der *Cystoviridae*-Familie stammt und wobei die Polymerase in der Lage ist, RNA-Synthese in Anwesenheit von ssRNA-, dsRNA-, ssDNA- und dsDNA-Substraten zu katalysieren.

**2.** Protein gemäß Anspruch 1, wobei das Protein aus Φ6-verwandten Bacteriophagen stammt.

**3.** Protein gemäß Anspruch 1 oder 2, wobei das Protein aus Φ6, Φ7, Φ8, Φ9, Φ10, Φ11, Φ12, Φ13 oder Φ14 stammt.

**4.** Protein gemäß einem der Ansprüche 1 bis 3, wobei das Protein P2 oder eine veränderte oder eine genetisch modifizierte Form von P2 ist, welches in isolierter Form nicht von anderen Proteinen unterstützt eine unspezifische, Primer-unabhängige Fähigkeit zur RNA-Synthese *in vitro* hat.

**5.** Protein gemäß einem der Ansprüche 1 bis 4 ausgewählt aus der Gruppe umfassend:

a) ein Protein codiert von einer Nucleinsäuresequenz, welche eine Nucleinsäuresequenz von SEQ ID NO:1 hat;
b) ein Protein codiert von einer Nucleinsäuresequenz, die ein Polypeptid codiert, welches eine Aminosäuresequenz von SEQ ID NO:8 hat; und
c) eine Aminosäuresequenz, die mindestens 80% Identität zu der Aminosäuresequenz SEQ ID NO:8 aufweist.

**6.** Protein gemäß einem der Ansprüche 1 bis 5, wobei das Protein die Aminosäuresequenz SEQ ID NO:8 umfasst.

**7.** Verfahren zur Herstellung des Proteins gemäß einem der Ansprüche 1 bis 6 umfassend die Schritte:

(a) Züchten von Wirtszellen enthaltend eine Nucleinsäuresequenz, die das Protein gemäß einem der Ansprüche 1 bis 6 codiert, in einem Kulturmedium zum Exprimieren des Proteins in löslicher Form;
(b) Gewinnen des Proteins aus dem Wirt oder dem Kulturmedium;
(c) Aufreinigen und Isolieren des Proteins von anderen Proteinen; und gegebenenfalls
(d) Prüfen der RNA-synthetisierenden Aktivität des Proteins.

**8.** Verfahren zur Isolierung und Aufreinigung des Proteins gemäß Anspruch 7, wobei das Verfahren die weiteren Schritte umfasst:

a) Aufschließen der Wirtszellen in einem Puffer, um ein Zelllysat zu erhalten;
b) Klären des Lysats durch Zentrifugation;
c) Aufreinigen des Proteins unter Verwendung von mindestens einem Schritt, mehr bevorzugt zwei Schritten Affinitätschromatographie;
d) weiteres Aufreinigen des Proteins unter Verwendung von mindestens einem Schritt Ionenaustauschchromatographie, um eine Fraktion zu erhalten, die im Wesentlichen frei von Nuclease- und Proteaseaktivitäten ist.

**9.** Verfahren zur Herstellung von RNA *in vitro,* umfassend die Schritte:

- Bereitstellen von ssRNA- oder dsRNA-Substrat;
- Inkontaktbringen des ssRNA- oder dsRNA-Substrats mit dem Protein gemäß einem der Ansprüche 1 bis 6 in isolierter Form unter Bedingungen, die ausreichend für RNA-Synthese sind; und
- Gewinnen der neu gebildeten RNA-Art aus dem Reaktionsgemisch.

**10.** Verfahren gemäß Anspruch 9, wobei die neu gebildete RNA-Art dsRNA ist.

**11.** Verfahren gemäß Anspruch 9 oder 10, umfassend folgende Schritte zum Amplifizieren von RNA *in vitro:*

(a) Bereitstellen des RNA-Substrats;
(b) Inkontaktbringen des RNA-Substrats mit dem Protein gemäß einem der Ansprüche 1 bis 6 in isolierter Form unter Bedingungen, die sowohl für RNA-Replikation als auch für RNA-Transkription ausreichend sind; und
(c) Gewinnen eines Gemisches aus neu gebildeter amplifizierter RNA aus dem Reaktionsgemisch.

**12.** Verfahren gemäß Anspruch 11, umfassend die Schritte:

(a) Bereitstellen von ssRNA-Substrat;
(b) Replizieren des ssRNA-Substrats mit dem Protein gemäß einem der Ansprüche 1 bis 6 in isolierter Form, um dsRNA zu bilden;
(c) Transkribieren der dsRNA mit dem Protein gemäß einem der Ansprüche 1 bis 6, um ssRNA zu erhalten; und
(d) Wiederholen der Amplifikationsschritte bis eine ausreichende Menge an RNA-Synthese-Produkten erhalten wurde.

**13.** Verfahren gemäß Anspruch 9 oder 10, umfassend die Schritte:

(a) Bereitstellen des ssRNA-Substrats durch Transkribieren einer DNA-Matrize mit einer DNA-abhängigen RNA-Polymerase; und
(b) Replizieren des ssRNA-Substrats mit dem Protein gemäß einem der Ansprüche 1 bis 6 in isolierter Form, um dsRNA zu bilden.

**14.** Verfahren gemäß Anspruch 13, weiter umfassend die folgenden Schritte zum Amplifizieren von RNA *in vitro*:

(c) Transkribieren der dsRNA mit dem Protein gemäß einem der Ansprüche 1 bis 6 in isolierter Form, um ssRNA zu erhalten; und
(d) Wiederholen der Amplifikationsschritte bis eine ausreichende Menge an RNA-Synthese-Produkten erhalten wurde.

**15.** Verfahren gemäß Anspruch 13 oder 14, wobei die DNA-abhängige RNA-Polymerase von einem Bacteriophagen, stammt, der bevorzugt ausgewählt ist aus der Gruppe umfassend T7-, T3- und SP6-Bacteriophagen.

**16.** Verfahren gemäß einem der Ansprüche 13 bis 15, wobei Schritte (a) und (b) gleichzeitig oder nacheinander in dem gleichen Reaktionsgefäß durchgeführt werden.

**17.** Verfahren zur Stabilisierung von Nucleinsäuren, umfassend die Schritte:

(a) Bereitstellen von einzelsträngigem Nucleinsäuresubstrat;
(b) Inkontaktbringen des einzelsträngigen Nucleinsäuresubstrats mit dem Protein gemäß einem der Ansprüche 1 bis 6 in isolierter Form unter Bedingungen, die ausreichend für die RNA-Synthese sind, um mindestens einen Teil des einzelsträngigen Nucleinsäuresubstrats in die doppelsträngige Nucleinsäureform umzuwandeln;

(c) Gewinnen der gesamten Nucleinsäuren aus dem Reaktionsgemisch; und
(d) Inkontaktbringen der gesamten Nucleinsäuren mit einem Nuclease oder Nucleasen enthaltenden Ansatz, die selektiv einzelsträngige Nucleinsäuren, nicht aber doppelsträngige Nucleinsäuren abbauen; und
(e) Gewinnen der doppelsträngigen Nucleinsäuren, die erhöhte Stabilität gegenüber den Abbau durch Nucleasen aufweisen.

**18.** Verfahren zur Herstellung von RNA *in vitro,* umfassend die Schritte:

(a) Bereitstellen eines ssDNA- oder dsDNA-Substrats;
(b) Inkontaktbringen des ssDNA- oder dsDNA-Substrats mit dem Protein gemäß einem der Ansprüche 1 bis 6 in isolierter Form unter Bedingungen, die ausreichend für die RNA-Synthese sind; und
(c) Gewinnen der neu gebildeten Nucleinsäureart aus dem Reaktionsgemisch.

**19.** Verfahren gemäß Anspruch 18, wobei die neu gebildete Nucleinsäureart Duplexe bestehend aus DNA- und RNA-Matrizen-Replicas umfasst.

**20.** Verfahren gemäß einem der Ansprüche 9 bis 19, wobei das einzelsträngige oder doppelsträngige Nucleinsäuresubstrat linear ist.

**21.** Verfahren gemäß einem der Ansprüche 9 bis 20, wobei das Gemisch für die RNA-Synthese mindestens ein Nucleosidtriphosphat enthält, das mit einem radioaktiven Isotop markiert ist oder das chemisch modifiziert ist.

**22.** Verfahren zur Herstellung von RNA *in vitro,* umfassend die Schritte:

- Bereitstellen von RNA- oder DNA-Substrat;
- Inkontaktbringen des RNA- oder DNA-Substrats mit dem Protein gemäß einem der Ansprüche 1 bis 6 in isolierter Form unter Bedingungen, die ausreichend für die RNA-Synthese sind, in einem Gemisch umfassend: Nucleinsäuresubstrat, Protein gemäß einem der Ansprüche 1 bis 6, Nucleosidtriphosphate und gegebenenfalls Puffer, Ammoniumacetat, DTT, PEG, $Mg^{2+}$-Ionen, $Mn^{2+}$-Ionen und/oder BSA; und
- Inkubieren des Reaktionsgemisches bei einer Temperatur, die ausreichend für die RNA-Synthese ist;
- Gewinnen der neu gebildeten Nucleinsäureart aus dem Reaktionsgemisch.

**23.** Verfahren gemäß einem der Ansprüche 9 bis 22, wobei die RNA-Synthese initiiert wird vom 3'-Ende eines Primers, der komplämentär zu dem Nucleinsäuresubstrat ist.

**24.** Verfahren gemäß Anspruch 23, wobei der Primer einzelsträngige RNA oder DNA ist.

**25.** Kit zum Herstellen von RNA *in vitro* umfassend:

(a) ein Polymeraseprotein gemäß einem der Ansprüche 1 bis 6 in isolierter Form, und gegebenenfalls
(b) Zusätze, die notwendig sind für einen nachweisbaren Spiegel der RNA-Synthese.

**26.** Kit gemäß Anspruch 25, umfassend Nucleosidtriphosphate in Konzentrationen, die ausreichend für die RNA-Synthese sind.

**27.** Kit gemäß Anspruch 25 oder 26, wobei mindestens ein Nucleosidtriphosphat mit einem radioaktiven Isotop markiert ist oder chemisch modifiziert ist.

**28.** Kit gemäß einem der Ansprüche 25 bis 27, wobei das Kit zusätzlich einen Standard-Nucleinsäureansatz (oder Ansätze) enthält mit der charakteristischen Fähigkeit als Matrize (Matrizen) für die RNA-Synthese zu dienen.

**29.** Kit gemäß einem der Ansprüche 25 bis 28, das spezifisch verwendet wird zum Sequenzieren eines Nucleinsäuremoleküls und gegebenenfalls mindestens ein RNA-Synthese-Terminationsagens umfasst, welches die RNA-Synthese an einer spezifischen Nucleotidbase beendet.

**30.** Kit gemäß Anspruch 29, wobei das RNA-Synthese-Terminationsagens ein 3'-Desoxynucleosidtriphosphat oder ein funktionelles Derivat davon ist.

**31.** Verfahren zum Bestimmen der Nucleotidbasensequenz eines linearen Nucleinsäuremoleküls, umfassend die Schritte:

(a) Bereitstellen eines linearen Nucleinsäuremoleküls;
(b) Inkubieren des Nucleinsäuremoleküls unter Bedingungen, die ausreichend für die RNA-Synthese in einem Gemisch sind, umfassend:

- Protein gemäß einem der Ansprüche 1 bis 6 in isolierter Form;
- vier Nucleosidtriphosphate oder funktionelle Analoga davon; und
- mindestens eins von vier RNA-Synthese-Terminations-Agenzien, welche die RNA-Synthese an einer spezifischen Nucleotidbase beenden, wobei jedes Agens die RNA-Synthese an einer anderen Nucleotidbase beendet; und

(c) Auftrennen der fertig gestellten RNA-Produkte einer Inkubationsreaktion nach ihrer Größe, wobei mindestens ein Teil der Nucleotidbasensequenz des Nucleinsäuremoleküls bestimmt werden kann.

**32.** Verfahren gemäß Anspruch 31, wobei das Nucleinsäuremolekül einzelsträngige RNA oder DNA ist.

**33.** Verfahren gemäß Anspruch 31, wobei das Nucleinsäuremolekül doppelsträngige RNA oder DNA ist.

**34.** Verfahren gemäß einem der Ansprüche 31 bis 33, umfassend die Verwendung von mindestens einem der Nucleosidtriphosphate oder funktionellen Analoga davon, die modifiziert wurden, um einen nachweisbaren Marker zu enthalten.

**35.** Verfahren gemäß einem der Ansprüche 31 bis 34, umfassend die Verwendung von mindestens einem der RNA-Synthese-Terminationsagenzien, die modifiziert wurden, um einen nachweisbaren Marker zu enthalten.

**36.** Verfahren gemäß einem der Ansprüche 31 bis 35, wobei die RNA-Synthese-Terminationsagenzien 3'-Desoxynucleosidtriphosphate oder funktionelle Derivate davon sind.

**Revendications**

**1.** Protéine polymérase d'ARN isolée dépendant de l'ARN, dans laquelle la protéine a, sous une forme isolée non assistée par une quelconque autre protéine, une capacité non spécifique indépendante de l'amorce de synthèse de l'ARN *in vitro* lorsqu'en contact avec des substrats d'acide nucléique dans des conditions suffisantes, ladite protéine provenant d'un virus de la famille des *Cystoviridae* et ladite polymérase étant capable de catalyser la synthèse de l'ARN en présence de substrats d'ARNsb, ARNdb, ADNsb et ADNdb.

**2.** Protéine selon la revendication 1, dans laquelle la protéine provient de bactériophages apparentés à Φ6.

**3.** Protéine selon la revendication 1 ou 2, dans laquelle la protéine provient de Φ6, Φ7, Φ8, Φ9, Φ10, Φ11, Φ12, Φ13 ou Φ14.

**4.** Protéine selon l'une quelconque des revendications 1 à 3, dans laquelle la protéine est P2 ou une forme altérée ou génétiquement modifiée de P2, ayant, sous une forme isolée non assistée par une quelconque autre protéine, une capacité non spécifique indépendante de l'amorce de synthèse de l'ARN *in vitro.*

**5.** Protéine selon l'une quelconque des revendications 1 à 4, choisie dans le groupe constitué par :

a) une protéine codée par une séquence d'acide nucléique ayant une séquence d'acide nucléique SEQ ID No. 1 ;
b) une protéine codée par une séquence d'acide nucléique codant un polypeptide ayant une séquence d'acide aminé SEQ ID No. 8 ; et
c) une séquence d'acide aminé qui présente une identité d'au moins 80 % avec la séquence d'acide aminé SEQ ID No. 8.

**6.** Protéine selon l'une quelconque des revendications 1 à 5, dans laquelle la protéine comprend la séquence d'acide aminé SEQ ID No. 8.

7. Procédé de production de la protéine selon l'une quelconque des revendications 1 à 6, comprenant les étapes consistant à :

(a) cultiver des cellules hôtes contenant une séquence d'acide nucléique codant pour la protéine selon l'une quelconque des revendications 1 à 6 dans un milieu de culture pour exprimer ladite protéine sous une forme soluble ;
(b) récupérer la protéine contenue dans l'hôte ou le milieu de culture ;
(c) purifier et isoler la protéine des autres protéines ;
et éventuellement
(d) doser l'activité de synthèse de l'ARN de ladite protéine.

8. Procédé d'isolement et de purification de la protéine selon la revendication 7, dans lequel le procédé comprend les étapes supplémentaires consistant à :

a) rompre les cellules hôtes dans un tampon pour obtenir un lysat de cellules ;
b) clarifier ledit lysat par centrifugation ;
c) purifier la protéine en utilisant au moins une étape, plus préférablement deux étapes de chromatographie d'affinité ;
d) davantage purifier la protéine en utilisant au moins une étape de chromatographie par échange d'ions pour obtenir une fraction qui est essentiellement exempte d'activités nucléase et protéase.

9. Procédé de production d'ARN *in vitro,* comprenant les étapes consistant à :

- fournir un substrat d'ARNsb ou d'ARNdb ;
- mettre en contact ledit substrat d'ARNsb ou d'ARNdb avec la protéine selon l'une quelconque des revendications 1 à 6 sous forme isolée dans des conditions suffisantes pour la synthèse de l'ARN ; et
- récupérer l'espèce d'ARN nouvellement produite contenue dans le mélange réactionnel.

10. Procédé selon la revendication 9, dans lequel ladite nouvelle espèce d'ARN nouvellement produite est l'ARNdb.

11. Procédé selon la revendication 9 ou 10, comprenant les étapes suivantes d'amplification d'ARN *in vitro* consistant à :

(a) fournir ledit substrat d'ARN ;
(b) mettre en contact ledit substrat d'ARN avec la protéine selon l'une quelconque des revendications 1 à 6 sous une forme isolée dans des conditions suffisantes pour à la fois la réplication de l'ARN et la transcription de l'ARN ; et
(c) récupérer un mélange de l'ARN amplifié nouvellement produit contenu dans le mélange réactionnel.

12. Procédé selon la revendication 11, comprenant les étapes consistant à :

(a) fournir un substrat d'ARNsb ;
(b) répliquer ledit substrat d'ARNsb avec la protéine selon l'une quelconque des revendications 1 à 6 sous une forme isolée pour former l'ARNdb ;
(c) transcrire ledit ARNdb avec la protéine selon l'une quelconque des revendications 1 à 6 pour obtenir l'ARNsb ; et
(d) répéter les étapes d'amplification jusqu'à l'obtention d'une quantité suffisante de produits de synthèse de l'ARN.

13. Procédé selon la revendication 9 ou 10, comprenant les étapes consistant à :

(a) fournir un substrat d'ARNsb par transcription d'une matrice d'ADN avec une polymérase d'ARN dépendant de l'ADN ; et
(b) répliquer ledit substrat d'ARNsb avec la protéine selon l'une quelconque des revendications 1 à 6 sous une forme isolée pour former l'ARNdb.

14. Procédé selon la revendication 13, comprenant en outre les étapes suivantes d'amplification d'ARN *in vitro* consistant à :

(c) transcrire ledit ARNdb avec la protéine selon l'une quelconque des revendications 1 à 6 sous une forme isolée pour obtenir l'ARNsb ; et
(d) répéter les étapes d'amplification jusqu'à l'obtention d'une quantité suffisante de produits de synthèse de l'ARN.

**15.** Procédé selon la revendication 13 ou 14, dans lequel ladite polymérase d'ARN dépendant de l'ADN est dérivée d'un bactériophage, de préférence choisi dans le groupe comprenant les bactériophages T7, T3 et SP6.

**16.** Procédé selon l'une quelconque des revendications 13 à 15, dans lequel les étapes (a) et (b) sont réalisées en même temps ou séquentiellement dans le même récipient réactionnel.

**17.** Procédé de stabilisation des acides nucléiques, comprenant les étapes consistant à :

(a) fournir un substrat d'acide nucléique simple brin ;
(b) mettre en contact ledit substrat d'acide nucléique simple brin avec la protéine selon l'une quelconque des revendications 1 à 6 sous une forme isolée dans des conditions suffisantes pour la synthèse de l'ARN afin de convertir au moins une partie du substrat d'acide nucléique simple brin en la forme d'acide nucléique double brin ;
(c) récupérer tous les acides nucléiques contenus dans le mélange réactionnel ; et
(d) mettre en contact tous lesdits acides nucléiques avec une préparation contenant une nucléase ou des nucléases dégradant de manière sélective les acides nucléiques simple brins mais pas les acides nucléiques double brin ; et
(e) récupérer les acides nucléiques double brin montrant une stabilité accrue vis-à-vis de la dégradation par les nucléases.

**18.** Procédé de production d'ARN *in vitro,* comprenant les étapes consistant à :

(a) fournir un substrat d'ADNsb ou d'ADNdb ;
(b) mettre en contact ledit substrat d'ADNsb ou d'ADNdb avec la protéine selon l'une quelconque des revendications 1 à 6 sous une forme isolée dans des conditions suffisantes pour la synthèse de l'ARN ; et
(c) récupérer la nouvelle espèce d'acide nucléique nouvellement produite contenue dans le mélange réactionnel.

**19.** Procédé selon la revendication 18, dans lequel l'espèce d'acide nucléique nouvellement produite comprend des duplexes constitués d'une réplique d'ARN et d'ADN de la matrice.

**20.** Procédé selon l'une quelconque des revendications 9 à 19, dans lequel le substrat d'acide nucléique simple brin ou double brin est linéaire.

**21.** Procédé selon l'une quelconque des revendications 9 à 20, dans lequel le mélange pour la synthèse de l'ARN comprend au moins un triphosphate de nucléoside marqué par un isotope radioactif ou qui est chimiquement modifié.

**22.** Procédé de production d'ARN *in vitro*, comprenant les étapes consistant à :

- fournir un substrat d'ARN ou d'ADN ;
- mettre en contact ledit substrat d'ARN ou d'ADN avec la protéine selon l'une quelconque des revendications 1 à 6 sous une forme isolée dans des conditions suffisantes pour la synthèse de l'ARN dans un mélange comprenant : un substrat d'acide nucléique, une protéine selon l'une quelconque des revendications 1 à 6, des triphosphates de nucléoside, et éventuellement un tampon, de l'acétate d'ammonium, du DTT, du PEG, des ions $Mg^{2+}$, des ions $Mn^{2+}$ et/ou de la BSA ; et
- incuber le mélange réactionnel à une température suffisante pour la synthèse de l'ARN ;
- récupérer l'espèce d'acide nucléique nouvellement produite contenue dans le mélange réactionnel.

**23.** Procédé selon l'une quelconque des revendications 9 à 22, dans lequel la synthèse de l'ARN est initiée à partir de l'extrémité 3' d'une amorce complémentaire du substrat d'acide nucléique.

**24.** Procédé selon la revendication 23, dans lequel ladite amorce est de l'ARN ou ADN simple brin.

**25.** Kit de production d'ARN *in vitro* comprenant :

(a) une protéine polymérase selon l'une quelconque des revendications 1 à 6 sous une forme isolée ; et éventuellement
(b) des additifs nécessaires pour un niveau détectable de synthèse de l'ARN.

**26.** Kit selon la revendication 25, comprenant des triphosphates de nucléoside à des concentrations suffisantes pour la synthèse de l'ARN.

**27.** Kit selon la revendication 25 ou 26, dans lequel au moins un triphosphate de nucléoside est marqué par un isotope radioactif ou est chimiquement modifié.

**28.** Kit selon l'une quelconque des revendications 25 à 27, dans lequel le kit comprend en outre une préparation (ou des préparations) standard d'acide nucléique avec une capacité **caractérisée** servant de matrice (ou matrices) pour la synthèse de l'ARN.

**29.** Kit selon l'une quelconque des revendications 25 à 28, spécifiquement utilisé pour le séquençage d'une molécule d'acide nucléique et comprenant éventuellement au moins un agent de fin de synthèse de l'ARN qui termine la synthèse de l'ARN au niveau d'une base nucléotide spécifique.

**30.** Kit selon la revendication 29, dans lequel ledit agent de fin de synthèse de l'ARN est un triphosphate de 3'-désoxynucléoside ou un dérivé fonctionnel de celui-ci.

**31.** Procédé de détermination de la séquence de base nucléotide d'une molécule d'acide nucléique linéaire, comprenant les étapes consistant à :

(a) fournir une molécule d'acide nucléique linéaire ;
(b) incuber ladite molécule d'acide nucléique dans des conditions suffisantes pour la synthèse de l'ARN dans un mélange comprenant :

- une protéine selon l'une quelconque des revendications 1 à 6 sous une forme isolée ;
- quatre triphosphates de nucléoside ou analogues onctionnels de ceux-ci ; et
- au moins un des quatre agents de fin de synthèse de l'ARN qui termine la synthèse de l'ARN au niveau d'une base nucléotide spécifique,
dans lequel chacun desdits agents termine la synthèse de l'ARN au niveau d'une base nucléotide différente ; et

(c) séparer les produits d'ARN terminés contenus dans la réaction d'incubation selon leur taille, au moins une partie de la séquence de base nucléotide de ladite molécule d'acide nucléique pouvant être déterminée.

**32.** Procédé selon la revendication 31, dans lequel ladite molécule d'acide nucléique est de l'ARN ou ADN simple brin.

**33.** Procédé selon la revendication 31, dans lequel ladite molécule d'acide nucléique est de l'ARN ou ADN double brin.

**34.** Procédé selon l'une quelconque des revendications 31 à 33, comprenant l'utilisation d'au moins un desdits triphosphates de nucléoside ou analogues fonctionnels de ceux-ci modifié pour contenir un marqueur détectable.

**35.** Procédé selon l'une quelconque des revendications 31 à 34, comprenant l'utilisation d'au moins un desdits agents de fin de synthèse de l'ARN modifié pour contenir un marqueur détectable.

**36.** Procédé selon l'une quelconque des revendications 31 à 35, dans lequel lesdits agents de fin de synthèse de l'ARN sont les triphosphates de 3'-désoxynucléoside ou des dérivés fonctionnels de ceux-ci.

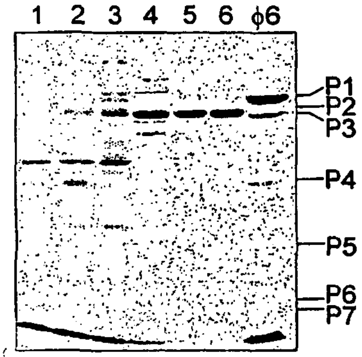


Fig. 1A

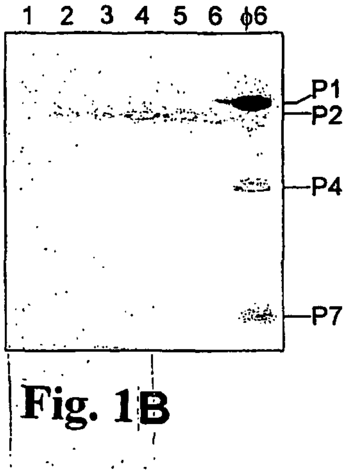


Fig. 1B

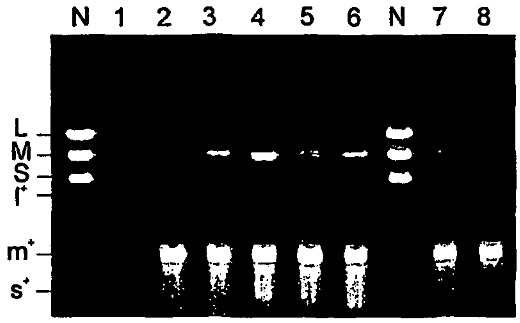


Fig. 2A

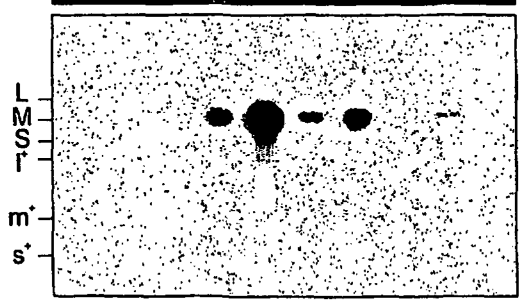


Fig. 2B

| | 1 | 2 | 3 | 4 | 5 | 6 | 7 | 8 |
|---|---|---|---|---|---|---|---|---|
| m+RNA, pmol | - | 1.3 | 1.3 | 1.3 | 1.3 | 1.3 | 1.3 | 1.3 |
| P2, pmol | 15 | - | 1.5 | 15 | 15 | 15 | - | - |
| P2-CBA, µl | - | - | - | - | - | - | 0.1 | - |
| mock-CBA, µl | - | - | - | - | - | - | - | 0.1 |
| (A,G)TP, mM | 1 | 1 | 1 | 1 | 1 | 0.2 | 1 | 1 |
| $MnCl_2$, mM | 1 | 1 | 1 | 1 | - | 1 | 1 | 1 |

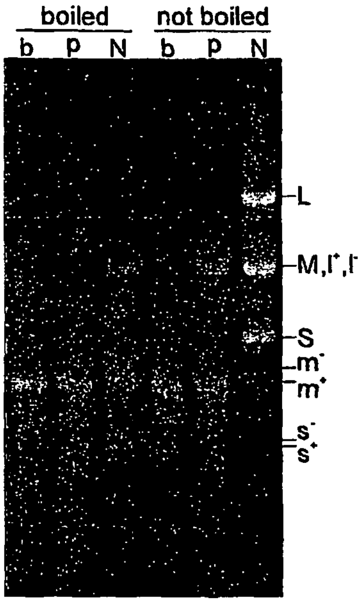


Fig. 3A

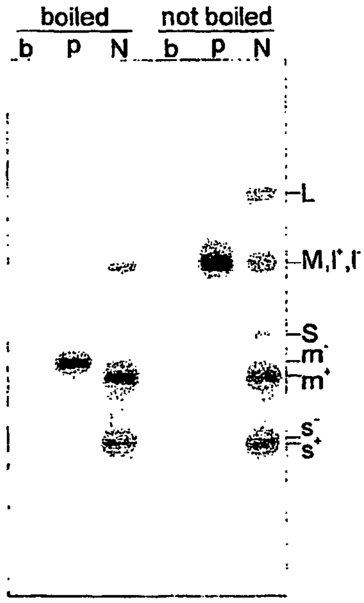


Fig. 3B

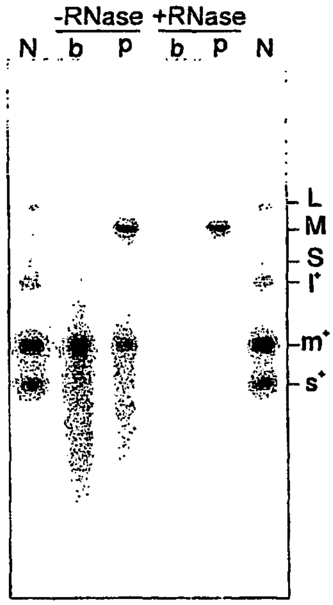


Fig. 3C

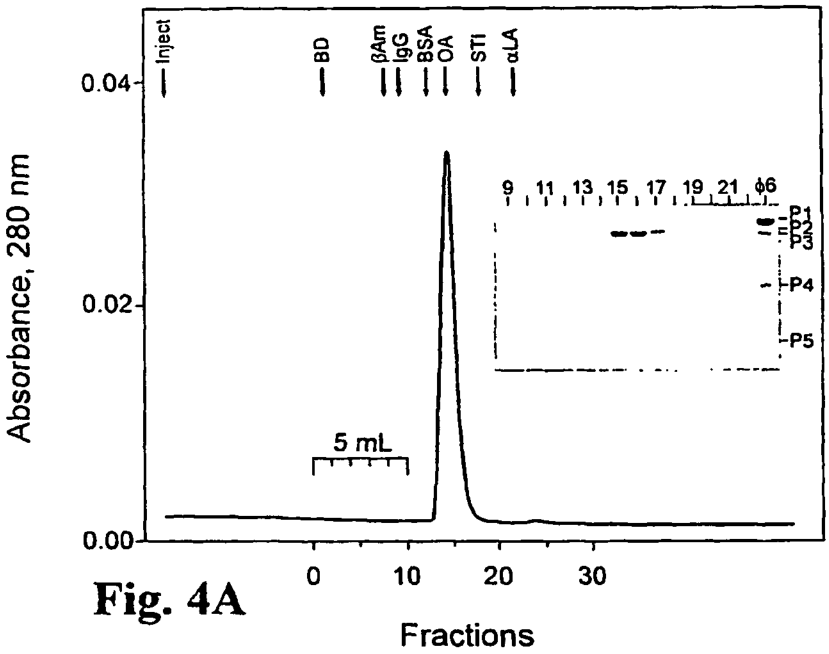
Inject
BD
βAm
IgG
BSA
OA
STI
αLA
0.04
0.02
0.00
Absorbance, 280 nm
5 mL
0
10
20
30
Fractions
9
11
13
15
17
19
21
φ6
P1
P2
P3
P4
P5

Fig. 4A

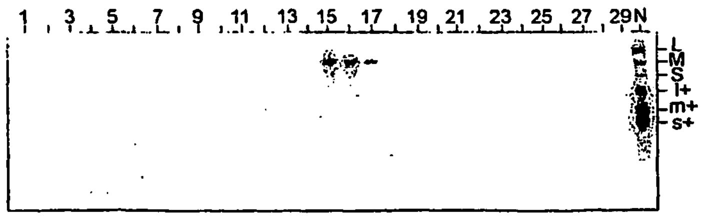
1
3
5
7
9
11
13
15
17
19
21
23
25
27
29
N
L
M
S
l+
m+
s+

Fig. 4B

# Fig. 5A

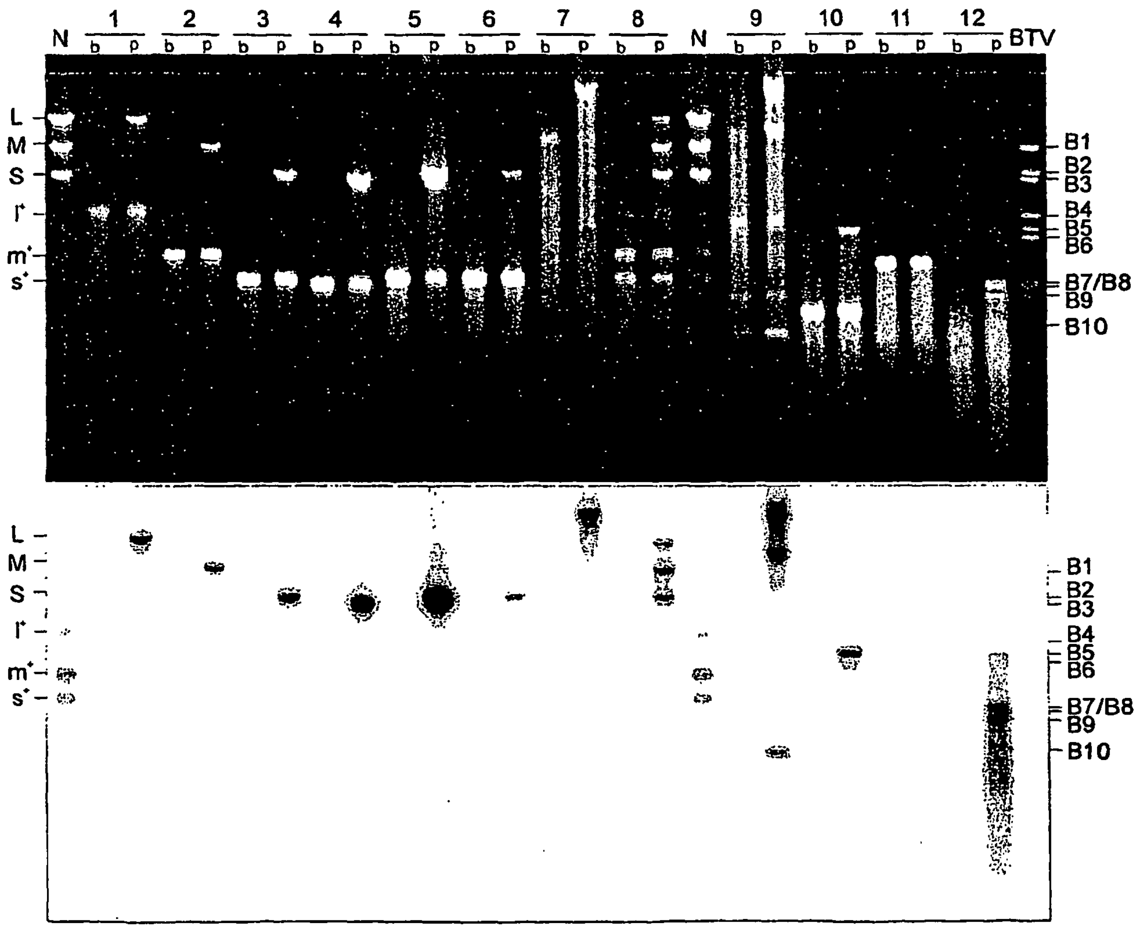
N 1 2 3 4 5 6 7 8 N 9 10 11 12 BTV
b p b p b p b p b p b p b p b p b p b p b p b p
L
M
S
l*
m*
s*
B1
B2
B3
B4
B5
B6
B7/B8
B9
B10

# Fig. 5B

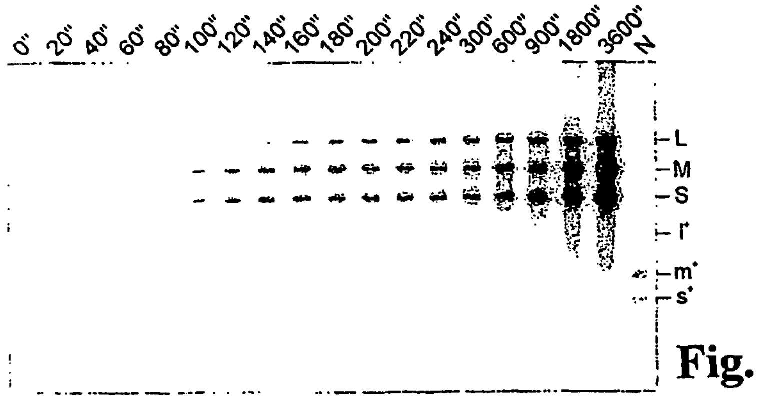


Fig. 6A

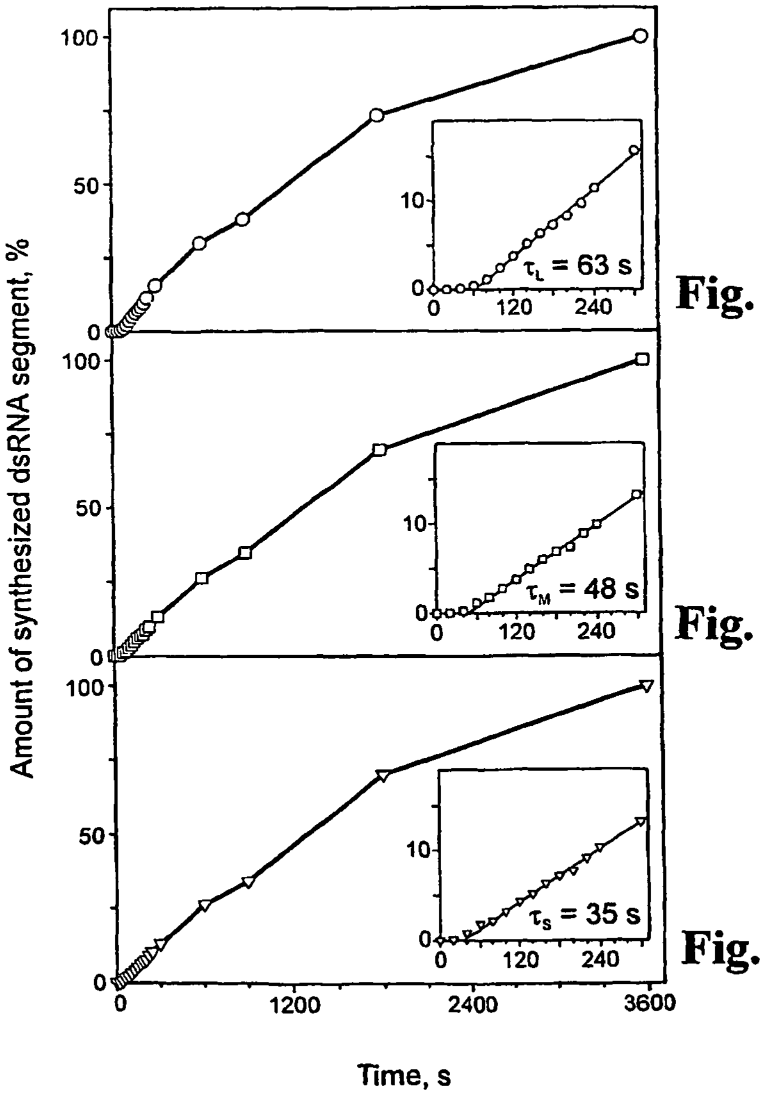


Fig. 6B

Fig. 6C

Fig. 6D

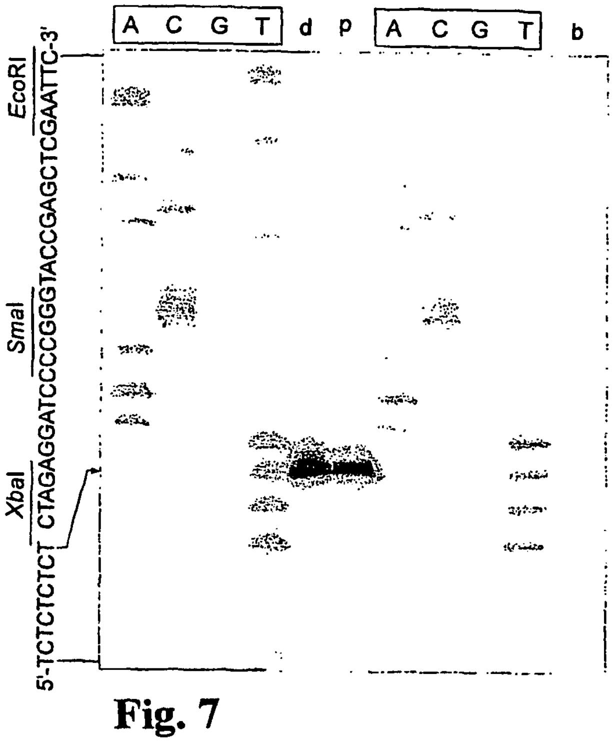


Fig. 7

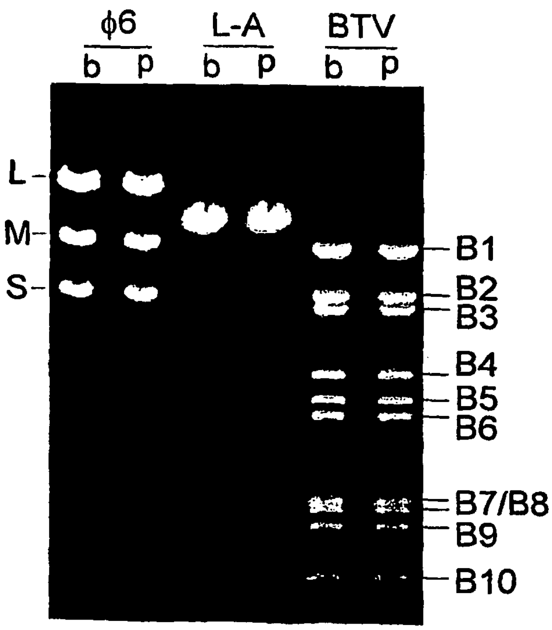


Fig. 8A

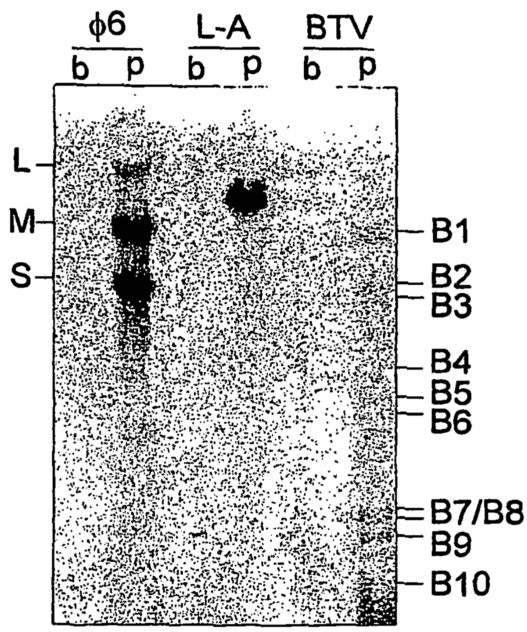


Fig. 8B

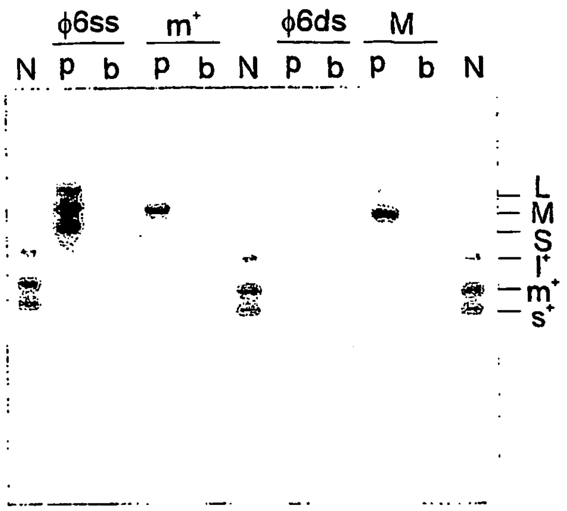


Fig. 9A

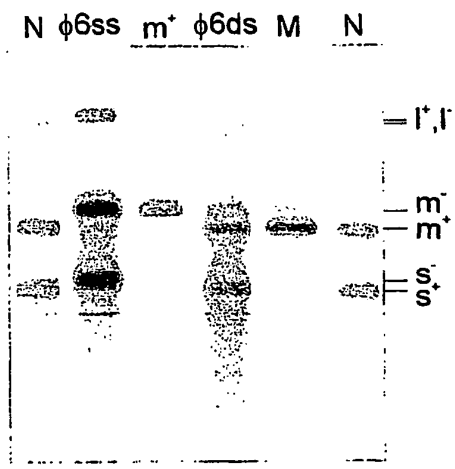


Fig. 9B

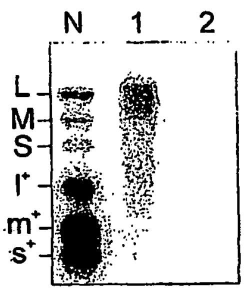


Fig. 10A

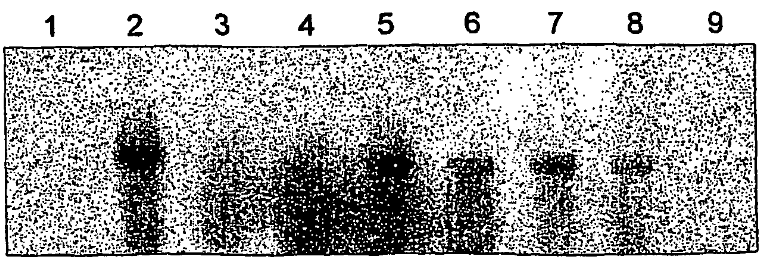


Fig. 10B

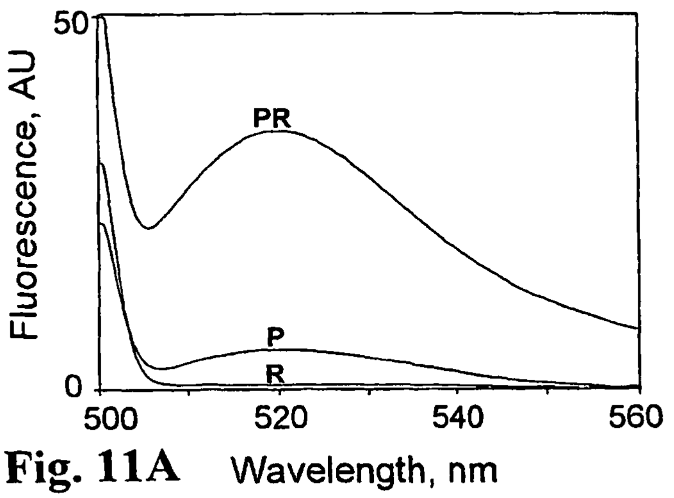


Fig. 11A

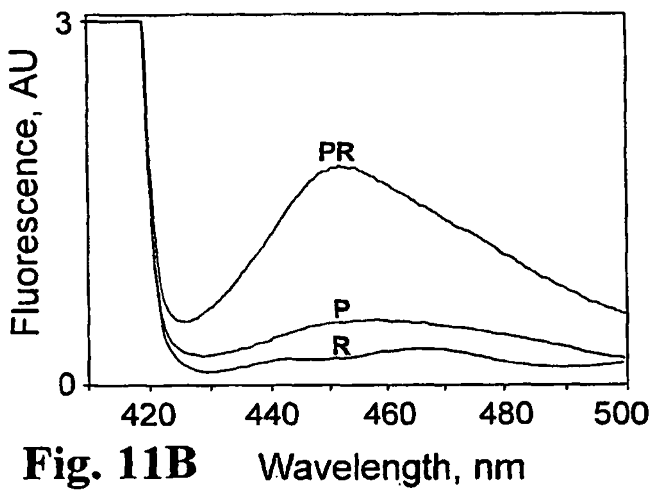


Fig. 11B

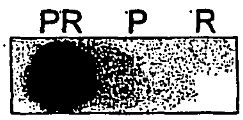


Fig. 11C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5631129 A
- US 5854037 A
- US 5614403 A
- US 5795715 A
- US 5173411 A

### Non-patent literature cited in the description


- **Makeyev ; Bamford.** *EMBO J.,* 2000, vol. 19, 6275-6284
- **Axelrod,V.D. ; Kramer,F.R.** Transcription from bacteriophage T7 and SP6 RNA polymerase promoters in the presence of 3'-deoxyribonucleoside 5'-triphosphate chain terminators. *Biochemistry,* 1985, vol. 24, 5716-5723
- Isolation, purification, and function of assembly intermediates and subviral particles of bacteriophages PRD1 and ϕ6. **Bamford,D.H. ; Ojala,P.M ; Frilander,M. ; Walin,L. ; Bamford,J.K.H.** Methods in Molecular Genetics. Academic Press, 1995, vol. 6, 455-474
- **Blumenthal,T.** Qβ replicase template specificity: different templates require different GTP concentrations for initiation. *Proc. Natl. Acad. Sci. USA,* 1980, vol. 77, 2601-2605
- **Bruenn,J.A.** Relationships among the positive strand and double-strand RNA viruses as viewed through their RNA-dependent RNA polymerases. *Nucleic Acids Res,* 1991, vol. 19, 217-226
- **Bruenn,J.A.** A closely related group of RNA-dependent RNA polymerases from double-stranded RNA viruses. *Nucleic Acids Res.,* 1993, vol. 21, 5667-5669
- **Brewer,G. ; Murray,E. ; Staeben,M.** RNase ONE: Advantages for nuclease protection assays. *Promega Notes Magazine,* 1992, vol. 38, 1-7
- **Butcher,S.J. ; Dokland,T. ; Ojala,P.M. ; Bamford,D.H. ; Fuller,S.D.** Intermediates in the assembly pathway of the double-stranded RNA virus ϕ6. *EMBO J.,* 1997, vol. 16, 4477-4487
- **Casini,G. ; Qiao,X. ; Mindich,L.** Reconstitution of active replicase in procapsids of the segmented dsRNA bacteriophage ϕ6. *Virology,* 1994, vol. 204, 251-253
- **Cohen,J.** Ribonucleic acid polymerase activity in purified infectious pancreatic necrosis virus of trout. *Biochem. Biophys. Res. Commun.,* 1975, vol. 62, 689-695
- **Chalfie,M. ; Tu,Y. ; Euskirchen,G. ; Ward,W.W. ; PrasherD.C.** green fluorescent protein as a marker for gene expression. *Science,* 1994, vol. 263, 802-805
- **Chen,D. ; Zeng,C.Q. ; Wentz,M.J. ; Gorziglia,M. ; Estes,M.K. ; Ramig,R.F.** Template-dependent, in vitro replication of rotavirus RNA. *J. Virol.,* 1994, vol. 68, 7030-7039
- **de Haas, F. ; Paatero, A.O. ; Mindich, L. ; Bamford, D.H ; Fuller, S.D.** A symmetry mismatch at the site of RNA packaging in the polymerase complex of dsRNA bacteriophage ϕ6. *J. Mol. Biol.,* 1999, vol. 294, 357-372
- **Dreher,T.W. ; Hall,T.C.** Mutational analysis of the sequence and structural requirements in brome mosaic virus RNA for minus strand promoter activity. *J. Mol. Biol.,* 1988, vol. 201, 31-40
- **Edelhoch,H.** Spectroscopic determination of tryptophan and tyrosine in proteins. *Biochemistry,* 1967, vol. 6, 1948-1954
- **Fields,B.N. ; Knipe,D.M.** Fields Virology. Raven Press, 1990
- **Frilander,M. ; Gottlieb,P. ; Strassman,J. ; Bamford,D.H. ; Mindich,L.** Dependence of minus-strand synthesis on complete genomic packaging in the double-stranded RNA bacteriophage ϕ6. *J. Virol.,* 1992, vol. 66, 5013-5017
- **Frilander,M. ; Bamford,D.H.** In vitro packaging of the single-stranded RNA genomic precursors of the segmented double-stranded RNA bacteriophage ϕ6: the three segments modulate each other's packaging efficiency. *J. Mol. Biol.,* 1995, vol. 246, 418-428
- **Fujimura,T. ; Esteban,R. ; Wickner,R.B.** In vitro L-A double-stranded RNA synthesis in virus-like particles from Saccharomyces cerevisiae. *Proc. Natl. Acad. Sci. USA,* 1986, vol. 83, 4433-4437
- **Fujimura,T. ; Wickner,R.B.** Replicase of L-A virus-like particles of Saccharomyces cerevisiae. In vitro conversion of exogenous L-A and M1 single-stranded RNAs to double-stranded form. *J. Biol. Chem.,* 1988, vol. 263, 454-460

- **Fujimura,T. ; Wickner,R.B.** Reconstitution of template-dependent in vitro transcriptase activity of a yeast double-stranded RNA virus. *J. Biol. Chem.,* 1989, vol. 264, 10872-10877
- **Gottlieb,P. ; Metzger,S. ; Romantschuk,M. ; Carton,J. ; Strassman,J. ; Bamford,D.H. ; Kalkkinen,N. ; Mindich,L.** Nucleotide sequence of the middle dsRNA segment of bacteriophage ϕ6: placement of the genes of membrane-associated proteins. *Virology,* 1988, vol. 163, 183-190
- **Gottlieb,P. ; Strassman,J. ; Qiao,X.Y. ; Frucht,A. ; Mindich,L.** In vitro replication, packaging, and transcription of the segmented double-stranded RNA genome of bacteriophage ϕ6: studies with procapsids assembled from plasmid-encoded proteins. *J. Bacteriol.,* 1990, vol. 172, 5774-5782
- **Gottlieb,P. ; Strassman,J. ; Mindich,L.** Protein P4 of the bacteriophage ϕ6 procapsid has a nucleoside triphosphate-binding site with associated nucleoside triphosphate phosphohydrolase activity. *J. Virol.,* 1992, vol. 66, 6220-6222
- **Gottlieb,P. ; Strassman,J. ; Qiao,X. ; Frilander,M. ; Frucht,A. ; Mindich,L.** In vitro packaging and replication of individual genomic segments of bacteriophage ϕ6 RNA. *J. Virol.,* 1992, vol. 66, 2611-2616
- **Herring,A.J. ; Bevan,E.A.** Yeast virus-like particles possess a capsid-associated single-stranded RNA polymerase. *Nature,* 1977, vol. 268, 464-466
- Reproduction of the reoviridae. **Joklik,W.K.** Comprehensive Virology. Plenim Press, 1974, vol. 2, 231-334
- **Juuti,J.T. ; Bamford,D.H.** RNA binding, packaging and polymerase activities of the different incomplete polymerase complex particles of dsRNA bacteriophage ϕ6. *J. Mol. Biol.,* 1995, vol. 249, 545-554
- **Juuti,J.T. ; Bamford,D.H.** Protein P7 of phage ϕ6 RNA polymerase complex, acquiring of RNA packaging activity by in vitro assembly of the purified protein onto deficient particles. *J. Mol. Biol.,* 1997, vol. 266 (89), 1-900
- **Juuti,J.T. ; Bamford,D.H. ; Tuma,R. ; Thomas,G.J.Jr.** Structure and NTPase activity of the RNA-translocating protein (P4) of bacteriophage ϕ6. *J. Mol. Biol.,* 1998, vol. 279, 347-359
- **Koonin,E.V. ; Gorbalenya,A.E. ; Chumakov,K.M.** Tentative identification of RNA-dependent RNA polymerases of dsRNA viruses and their relationship to positive strand RNA viral polymerases. *FEBS Lett.,* 1989, vol. 252, 42-46
- **Macreadie,I.G. ; Azad,A.A.** Expression and RNA dependent RNA polymerase activity of birnavirus VP1 protein in bacteria and yeast. *Biochem. Mol. Biol. Int.,* 1993, vol. 30, 1169-1178
- **Makeyev,E.V. ; Kolb,V.A. ; Spirin,A.S.** Enzymatic activity of the ribosome-bound nascent polypeptide. *FEBS Lett.,* 1996, vol. 378, 166-170
- **McGraw,T. ; Mindich,L. ; Frangione,B.** Nucleotide sequence of the small double-stranded RNA segment of bacteriophage ϕ6: novel mechanism of natural translational control. *J. Virol.,* 1986, vol. 58, 142-151
- **Mindich,L. ; Nemhauser,I. ; Gottlieb,P. ; Romantschuk,M. ; Carton,J. ; Frucht,S. ; Strassman,J. ; Bamford,D.H. ; Kalkkinen,N.** Nucleotide sequence of the large double-stranded RNA segment of bacteriophage ϕ6: genes specifying the viral replicase and transcriptase. *J. Virol.,* 1988, vol. 62, 1180-1185
- **Mindich,L. ; Qiao,X. ; Onodera,S. ; Gottlieb,P. ; Frilander,M.** RNA structural requirements for stability and minus-strand synthesis of in the dsRNA bacteriophage ϕ6. *Virology,* 1994, vol. 202, 258-263
- **Mindich,L. ; Qiao,X. ; Qiao,J. ; Onodera,S. ; Romantschuk,M. ; Hoogstraten,D.** Isolation of additional bacteriophages with genomes of segmented double-stranded RNA. *J. Bacteriol.,* 1999, vol. 181, 4505-4508
- **Olkkonen,V.M. ; Bamford,D.H.** Quantitation of the adsorption and penetration stages of bacteriophage ϕ6 infection. *Virology,* 1989, vol. 171, 229-238
- **Olkkonen,V.M. ; Gottlieb,P. ; Strassman,J. ; Qiao,X.Y. ; Bamford,D.H. ; Mindich,L.** In vitro assembly of infectious nucleocapsids of bacteriophage ϕ6: Formation of a recombinant double-stranded RNA virus. *Proc. Natl. Acad. Sci. USA,* 1990, vol. 87, 9173-9177
- **Paatero,A.O. ; Syvaoja,J.E. ; Bamford,D.H.** Double-stranded RNA bacteriophage ϕ6 protein P4 is an unspecific nucleoside triphosphatase activated by calcium ions. *J. Virol.,* 1995, vol. 69, 6729-6734
- **Paatero,A.O. ; Mindich,L. ; Bamford,D.H.** Mutational analysis of the role of nucleoside triphosphatase P4 in the assembly of the RNA polymerase complex of bacteriophage ϕ6. *J. Virol.,* 1998, vol. 72, 10058-10065
- **Pagratis,N. ; Revel,H.R.** Detection of bacteriophage ϕ6 minus-strand RNA and novel mRNA isoconformers synthesized in vivo and in vitro, by strand-separating agarose gels. *Virology,* 1990, vol. 177, 273-280
- **Partridge,J.E. ; Van Etten,J.L. ; Burbank,D.E. ; Vidaver,A.K.** RNA polymerase associated with bacteriophage ϕ6 nucleocapsid. *J. Gen. Virol.,* 1979, vol. 43, 299-307
- **Patton,J.T.** Rotavirus VP1 alone specifically binds to the 3’ end of viral mRNA, but the interaction is not sufficient to initiate minus-strand synthesis. *J. Virol.,* 1996, vol. 70, 7940-7947

- **Patton,J.T. ; Jones,M.T. ; Kalbach,A.N. ; He,Y.W. ; Xiaobo,J.** Rotavirus RNA polymerase requires the core shell protein to synthesize the double-stranded RNA genome. *J. Virol.,* 1997, vol. 71, 9618-9626
- **Qiao,X. ; Qiao,J. ; Mindich,L.** Stochiometric packaging of the three genomic segments of double-stranded RNA bacteriophage ϕ6. *Proc. Natl. Acad. Sci. USA,* 1997, vol. 94, 4074-4079
- **Sambrook et al.** Molecular Cloning, A Laboratory Manual. Cold Spring Harbor Laboratory Press, 1989
- **Semancik,J.S. ; Vidaver,A.K. ; Van Etten,J.L.** Characterization of segmented double-helical RNA from bacteriophage ϕ6. *J. Mol. Biol.,* 1973, vol. 78, 617-625
- **Sharp,P.A.** RNAi and double-strand RNA. *Genes Dev.,* 1999, vol. 13, 139-141
- **Studier,F.W. ; Moffatt,B.A.** Use of bacteriophage T7 RNA polymerase to direct selective high-level expression of cloned genes. *J. Mol. Biol.,* 1986, vol. 189, 113-130
- **Urakawa,T. ; Ritter,D.G. ; Roy,P.** Expression of largest RNA segment and synthesis of VP1 protein of bluetongue virus in insect cells by recombinant baculovirus: association of VP1 protein with RNA polymerase activity. *Nucleic Acids Res.,* 1989, vol. 17, 7395-7401
- **Valenzuela,S. ; Pizarro,J. ; Sandino,A.M. ; Vasquez,M. ; Femandez,J. ; Hernandez,O. ; Patton,J. ; Spencer,E.** Photoaffinity labeling of rotavirus VP1 with 8-azido-ATP: identification of the viral RNA polymerase. *J. Virol.,* 1991, vol. 65, 3964-3967
- **Van Etten,J.L. ; Vidaver,A.K. ; Koski,RK. ; Semancik,J.S.** RNA polymerase activity associated with bacteriophage ϕ6. *J. Virol.,* 1973, vol. 12, 464-47 1
- **Van Etten,J.L. ; Vidaver,A.K. ; Koski,R.K. ; Bumett,J.P.** Base composition and hybridization studies of the three double-stranded RNA segments of bacteriophage ϕ6. *J. Virol.,* 1974, vol. 13, 1254-1262
- **Van Dijk,A.A. ; Frilander,M. ; Bamford,D.H.** Differentiation between minus- and plus-strand synthesis: polymerase activity of dsRNA bacteriophage phi 6 in an in vitro packaging and replication system. *Virology,* 1995, vol. 211, 320-323
- **Vidaver,A.K. ; Koski,R.K. ; Van Etten,J.L.** Bacteriophage ϕ6: a lipid-containing virus of Pseudomonas phaseolicola. *J. Virol.,* 1973, vol. 11, 799-805
- **Zeng,C.Q. ; Wentz,M.J. ; Cohen,J. ; Estes,M.K. ; Ramig,R.F.** Characterization and replicase activity of double-layered and single-layered rotavirus-like particles expressed from baculovirus recombinants. *J. Virol.,* 1996, vol. 70, 2736-2742